# EUROPEAN PATENT SPECIFICATION

(11) **EP 3 430 154 B1**
(45) Date of publication and mention of the grant of the patent: **11.11.2020**
(21) Application number: 17767301.9
(22) Date of filing: 14.03.2017
(51) Int. Cl.: C12P 19/30, C12P 19/34, C12Q 1/68

(54) **HYPER-THERMOSTABLE LYSINE-MUTANT SSDNA/RNA LIGASES**
HYPERTHERMOSTABILE LYSIN-MUTANTE SSDNA/RNA-LIGASEN
ADNSB/ARNSB LIGASES MUTANTES POUR LA LYSINE ET HYPER-THERMOSTABLES

(30) Priority: 14.03.2016 US 201662307658 P
(43) Date of publication of application: 23.01.2019
(73) Proprietor: Rgene, Inc., San Francisco, California 94107 (US)
(72) Inventor: ZHENG, Yu, Redwood City CA 94065 (US); HONG, Manqing, San Francisco California 94107 (US)
(74) Representative: Glawe, Delfs, Moll
(86) International application number: PCT/US2017/022232
(87) International publication number: WO 2017/160788

(56) References cited:
- EP-A1- 0 956 359
- US-A1- 2005 266 439
- US-A1- 2010 209 940
- US-A1- 2014 128 292
- US-A1- 2015 031 086
- US-B1- 6 280 998
- US-B1- 6 949 370
- ALEXANDER M ZHELKOVSKY ET AL: "Structure-function analysis of Methanobacterium thermoautotrophicum RNA ligase - engineering a thermostable ATP independent en", BMC MOLECULAR BIOLOGY, BIOMED CENTRAL LTD, GB, vol. 13, no. 1, 18 July 2012 (2012-07-18), page 24, XP021128041, ISSN: 1471-2199, DOI: 10.1186/1471-2199-13-24
- NAKATANI M ET AL: "A DNA LIGASE FROM A HYPERTHERMOPHILIC ARCHAEON WITH UNIQUE COFACTOR SPECIFICITY", JOURNAL OF BACTERIOLOGY, AMERICAN SOCIETY FOR MICROBIOLOGY, US, vol. 182, no. 22, 1 January 2000 (2000-01-01), pages 6424-6433, XP002974649, ISSN: 0021-9193, DOI: 10.1128/JB.182.22.6424-6433.2000
- KEPPETIPOLA NIROSHIKA ET AL: "Characterization of a thermophilic ATP-dependent DNA ligase from the euryarchaeon Pyrococcus horikoshii", JOURNAL OF BACTERIOLOGY, AMERICAN SOCIETY FOR MICROBIOLOGY, US, vol. 187, no. 20, 1 October 2005 (2005-10-01), pages 6902-6908, XP002414159, ISSN: 0021-9193, DOI: 10.1128/JB.187.20.6902-6908.2005
- Saiful Islam: "Efficient ligation of DNA on RNA templates using a mutated T4 DNA ligase", Master^s Thesis University of Uppsala, 1 January 2008 (2008-01-01), pages 1-21, XP055408645, Retrieved from the Internet: URL:http://www.ibg.uu.se/digitalAssets/164 /c_164781-l_3-k_islam-saiful-arbete-1336.p df [retrieved on 2017-09-21]
- LUO J ET AL: "Improving the fidelity of Thermus thermophilus DNA ligase", NUCLEIC ACIDS RESEARCH, INFORMATION RETRIEVAL LTD, vol. 24, no. 14, 1 August 1996 (1996-08-01), pages 3071-3078, XP002141352, ISSN: 0305-1048, DOI: 10.1093/NAR/24.15.3071

### SEQUENCE LISTING

<110> RGENE, INC.
<120> HYPER-THERMOSTABLE LYSINE-MUTANT ssDNA/RNA LIGASES
<130> RGENE-34782/WO-1/ORD
<150> US 62/307,658
   <151> 2016-03-14
<160> 32
<170> PatentIn version 3.5
<210> 1
   <211> 380
   <212> PRT
   <213> Thermococcus kodakarensis
<220>
   <221> misc_feature
   <222> (92)..(92)
   <223> Xaa can be any naturally occurring amino acid, except K
<400> 1
   Met Val Ser Ser Tyr Phe Arg Asn Leu Leu Leu Lys Leu Gly Leu Pro
   1               5                   10                  15
   Glu Glu Arg Leu Glu Val Leu Glu Gly Lys Gly Ala Leu Ala Glu Asp
               20                  25                  30
   Glu Phe Glu Gly Ile Arg Tyr Val Arg Phe Arg Asp Ser Ala Arg Asn
           35                  40                  45
   Phe Arg Arg Gly Thr Val Val Phe Glu Thr Gly Glu Ala Val Leu Gly
       50                  55                  60
   Phe Pro His Ile Lys Arg Val Val Gln Leu Glu Asn Gly Ile Arg Arg
   65                  70                  75                  80
   Val Phe Lys Asn Lys Pro Phe Tyr Val Glu Glu Xaa Val Asp Gly Tyr
                   85                  90                  95
   Asn Val Arg Val Val Lys Val Lys Asp Lys Ile Leu Ala Ile Thr Arg
               100                 105                 110
   Gly Gly Phe Val Cys Pro Phe Thr Thr Glu Arg Ile Glu Asp Phe Val
           115                 120                 125
   Asn Phe Asp Phe Phe Lys Asp Tyr Pro Asn Leu Val Leu Val Gly Glu
       130                 135                 140
   Met Ala Gly Pro Glu Ser Pro Tyr Leu Val Glu Gly Pro Pro Tyr Val
   145 150 155 160
   Lys Glu Asp Ile Glu Phe Phe Leu Phe Asp Ile Gln Glu Lys Gly Thr
   165 170 175
   Gly Arg Ser Leu Pro Ala Glu Glu Arg Tyr Arg Leu Ala Glu Glu Tyr
   180 185 190
   Gly Ile Pro Gln Val Glu Arg Phe Gly Leu Tyr Asp Ser Ser Lys Val
   195 200 205
   Gly Glu Leu Lys Glu Leu Ile Glu Trp Leu Ser Glu Glu Lys Arg Glu
   210 215 220
   Gly Ile Val Met Lys Ser Pro Asp Met Arg Arg Ile Ala Lys Tyr Val
   225 230 235 240
   Thr Pro Tyr Ala Asn Ile Asn Asp Ile Lys Ile Gly Ser His Ile Phe
   245 250 255
   Phe Asp Leu Pro His Gly Tyr Phe Met Gly Arg Ile Lys Arg Leu Ala
   260 265 270
   Phe Tyr Leu Ala Glu Asn His Val Arg Gly Glu Glu Phe Glu Asn Tyr
   275 280 285
   Ala Lys Ala Leu Gly Thr Ala Leu Leu Arg Pro Phe Val Glu Ser Ile
   290 295 300
   His Glu Val Ala Asn Gly Gly Glu Val Asp Glu Thr Phe Thr Val Arg
   305 310 315 320
   Val Lys Asn Ile Thr Thr Ala His Lys Met Val Thr His Phe Glu Arg
   325 330 335
   Leu Gly Val Lys Ile His Ile Glu Asp Ile Glu Asp Leu Gly Asn Gly
   340 345 350
   Tyr Trp Arg Ile Thr Phe Lys Arg Val Tyr Pro Asp Ala Thr Arg Glu
   355 360 365
   Ile Arg Glu Leu Trp Asn Gly Leu Ala Phe Val Asp
   370 375 380
<210> 2
   <211> 379
   <212> PRT
   <213> Pyrococcus yayanosii
<220>
   <221> misc_feature
   <222> (92)..(92)
   <223> Xaa can be any naturally occurring amino acid, except K
<400> 2
   Met Val Ser Ser His Phe Lys Glu Ile Leu Met Arg Leu Gly Leu Pro
   1               5                   10                  15
   Glu Asp Arg Ile Glu Val Leu Glu Ala Lys Gly Gly Ile Thr Glu Glu
               20                  25                  30
   Glu Phe Asp Gly Ile Arg Tyr Leu Arg Phe Lys Asp Ser Ala Arg Gly
           35                  40                  45
   Leu Arg Arg Gly Thr Val Val Phe Asp Glu Ala Asn Val Ile Leu Gly
       50                  55                  60
   Phe Pro His Ile Lys Arg Val Val Ser Leu Arg Ala Gly Val Met Arg
   65                  70                  75                  80
   Ile Phe Lys Arg Thr Pro Phe Tyr Val Glu Glu Xaa Val Asp Gly Tyr
                   85                  90                  95
   Asn Val Arg Val Ala Leu Val Ser Asp Arg Val Leu Ala Ile Thr Arg
               100                 105                 110
   Gly Gly Phe Val Cys Pro Phe Thr Thr Glu Arg Ile Leu Asp Phe Val
           115                 120                 125
   Pro Glu Glu Phe Phe Lys Asp Tyr Pro His Leu Val Leu Val Gly Glu
       130                 135                 140
   Met Ala Gly Pro Glu Ser Pro Tyr Leu Val Glu Gly Pro Pro Tyr Val
   145                 150                 155                 160
   Glu Glu Asp Ile Arg Phe Phe Leu Phe Asp Ile Gln Glu Lys Gly Thr
                   165                 170                 175
   Gly Lys Ser Leu Pro Val Gln Glu Arg Leu Lys Leu Ala Glu Glu Tyr
               180                 185                 190
   Gly Ile Pro His Val Lys Val Phe Gly Leu Tyr Thr Val Asp Arg Ile
           195                 200                 205
   Glu Asp Leu Tyr Asp Leu Ile Glu Arg Leu Ser Arg Glu Gly Arg Glu
       210                 215                 220
   Gly Val Val Met Lys Ser Pro Asp Met Lys Arg Val Val Lys Tyr Val
   225                 230                 235                 240
   Thr Pro Phe Ala Asn Val Asn Asp Val Lys Ile Gly Ala Lys Val Phe
                   245                 250                 255
   Phe Glu Leu Pro Pro Gly Tyr Phe Met Ser Arg Ile Met Arg Leu Ala
               260                 265                 270
   Phe Tyr Val Ala Glu Arg Arg Ile Lys Gly Glu Arg Phe Glu Glu Leu
           275                 280                 285
   Ala Arg Asn Leu Gly Lys Ala Leu Leu Glu Pro Phe Val Glu Ser Ile
       290                 295                 300
   Trp Asp Val Glu Gln Gly Asp Glu Ile Ala Glu Val Phe Arg Ile Arg
   305                 310                 315                 320
   Val Lys Arg Ile Glu Thr Ala Tyr Lys Met Val Thr His Phe Glu Arg
                   325                 330                 335
   Leu Gly Leu Asn Ile Lys Ile Glu Asp Ile Glu Glu Val Gly Gly Met
               340                 345                 350
   Trp Arg Ile Thr Phe Lys Arg Ala Tyr Asp Glu Ala Thr Arg Glu Ile
           355                 360                 365
   Arg Glu Leu Ile Gly Gly Arg Ala Phe Val Asp
       370                 375
<210> 3
   <211> 379
   <212> PRT
   <213> Pyrococcus horikoshii
<220>
   <221> misc_feature
   <222> (92)..(92)
   <223> Xaa can be any naturally occurring amino acid, except K
<220>
   <221> misc_feature
   <222> (107)..(107)
   <223> Xaa can be any naturally occurring amino acid, except K
<400> 3
   Met Val Ser Ser Lys Phe Lys Asp Ile Leu Tyr Arg Leu Gly Ile Pro
   1               5                   10                  15
   Glu Gly Lys Val Glu Asp Leu Glu Ala Arg Gly Gly Leu Val Glu Asp
               20                  25                  30
   Lys Phe Asp Asp Ile Lys Tyr Leu Arg Ile Arg Asn Ser Val Gly Lys
           35                  40                  45
   Leu Arg Arg Gly Thr Val Val Leu Asn Asp Lys Phe Ile Ile Leu Gly
       50                  55                  60
   Phe Pro His Ile Lys Arg Ile Val Asn Leu Lys Asn Gly Ile Lys Arg
   65                  70                  75                  80
   Thr Phe Lys Arg Gly Glu Phe Tyr Val Glu Glu Xaa Val Asp Gly Tyr
                   85                  90                  95
   Asn Val Arg Val Val Lys Phe Arg Gly Lys Xaa Leu Gly Ile Thr Arg
               100                 105                 110
   Gly Gly Phe Ile Cys Pro Phe Thr Thr Glu Arg Ile Ser Asp Phe Ile
           115                 120                 125
   Pro Glu Glu Phe Phe Lys Asp His Pro Asn Leu Ile Leu Val Gly Glu
       130                 135                 140
   Met Ala Gly Pro Glu Ser Pro Tyr Leu Val Glu Gly Pro Pro Tyr Val
   145                 150                 155                 160
   Lys Glu Asp Ile Gln Phe Phe Leu Phe Asp Ile Gln Glu Leu Gly Thr
                   165                 170                 175
   Gly Arg Ser Leu Pro Val Glu Glu Arg Leu Lys Ile Ala Glu Glu Tyr
               180                 185                 190
   Gly Ile Ser His Val Glu Val Phe Gly Lys Phe Thr Tyr Lys Asp Leu
           195                 200                 205
   Glu Glu Ile Tyr Glu Ile Val Glu Arg Leu Ser Arg Glu Gly Arg Glu
       210                 215                 220
   Gly Ile Val Met Lys Ser Pro Asp Met Arg Lys Met Val Lys Tyr Val
   225                 230                 235                 240
   Thr Pro Tyr Ala Asn Ile Asn Asp Ile Lys Ile Gly Ala Arg Val Phe
                   245                 250                 255
   Tyr Glu Leu Pro Pro Gly Tyr Phe Thr Ser Arg Ile Ser Arg Leu Ala
                   260                 265                 270
   Phe Tyr Ile Ala Glu Lys Arg Leu Arg Gly Glu Asn Phe Glu Glu Leu
           275                 280                 285
   Ala Lys Glu Leu Gly Lys Ala Leu Leu Gln Pro Leu Val Glu Ser Ile
       290                 295                 300
   His Asp Val Glu Gln Glu Asp Glu Ile Ala Glu Val Phe Lys Val Arg
   305                 310                 315                 320
   Val Lys Lys Ile Glu Thr Ala Tyr Lys Met Val Thr His Phe Glu Lys
                   325                 330                 335
   Leu Gly Leu Arg Ile Glu Ile Val Asp Ile Glu Glu Met Lys Gly Gly
               340                 345                 350
   Trp Arg Ile Thr Phe Lys Arg Leu Tyr Pro Asp Ala Thr Glu Glu Ile
           355                 360                 365
   Arg Glu Leu Ile Gly Gly Lys Ser Phe Val Asp
       370                 375
<210> 4
   <211> 382
   <212> PRT
   <213> Pyrococcus abyssi
<220>
   <221> misc_feature
   <222> (95)..(95)
   <223> Xaa can be any naturally occurring amino acid, except K
<400> 4
   Met Lys Glu Val Val Ser Ser Val Tyr Lys Glu Ile Leu Val Lys Leu
   1               5                   10                  15
   Gly Leu Thr Glu Asp Arg Ile Glu Thr Leu Glu Met Lys Gly Gly Ile
               20                  25                  30
   Ile Glu Asp Glu Phe Asp Gly Ile Arg Tyr Val Arg Phe Lys Asp Ser
           35                  40                  45
   Ala Gly Lys Leu Arg Arg Gly Thr Val Val Ile Asp Glu Glu Tyr Val
       50                  55                  60
   Ile Pro Gly Phe Pro His Ile Lys Arg Ile Ile Asn Leu Arg Ser Gly
   65                  70                  75                  80
   Ile Arg Arg Ile Phe Lys Arg Gly Glu Phe Tyr Val Glu Glu Xaa Val
                   85                  90                  95
   Asp Gly Tyr Asn Val Arg Val Val Met Tyr Lys Gly Lys Met Leu Gly
               100                 105                 110
   Ile Thr Arg Gly Gly Phe Ile Cys Pro Phe Thr Thr Glu Arg Ile Pro
           115                 120                 125
   Asp Phe Val Pro Gln Glu Phe Phe Lys Asp Asn Pro Asn Leu Ile Leu
       130                 135                 140
   Val Gly Glu Met Ala Gly Pro Glu Ser Pro Tyr Leu Val Glu Gly Pro
   145                 150                 155                 160
   Pro Tyr Val Lys Glu Asp Ile Gln Phe Phe Leu Phe Asp Val Gln Glu
                   165                 170                 175
   Ile Lys Thr Gly Arg Ser Leu Pro Val Glu Glu Arg Leu Lys Ile Ala
               180                 185                 190
   Glu Glu Tyr Gly Ile Asn His Val Glu Val Phe Gly Lys Tyr Thr Lys
           195                 200                 205
   Asp Asp Val Asp Glu Leu Tyr Gln Leu Ile Glu Arg Leu Ser Lys Glu
       210                 215                 220
   Gly Arg Glu Gly Ile Ile Met Lys Ser Pro Asp Met Lys Lys Ile Val
   225                 230                 235                 240
   Lys Tyr Val Thr Pro Tyr Ala Asn Ile Asn Asp Ile Lys Ile Gly Ala
                   245                 250                 255
   Arg Val Phe Tyr Glu Leu Pro Pro Gly Tyr Phe Thr Ser Arg Ile Ser
               260                 265                 270
   Arg Leu Ala Phe Tyr Leu Ala Glu Lys Arg Ile Lys Gly Glu Glu Phe
           275                 280                 285
   Glu Arg Val Ala Lys Glu Leu Gly Ser Ala Leu Leu Gln Pro Phe Val
       290                 295                 300
   Glu Ser Ile Phe Asp Val Glu Gln Glu Glu Asp Ile His Glu Leu Phe
   305                 310                 315                 320
   Lys Val Arg Val Lys Arg Ile Glu Thr Ala Tyr Lys Met Val Thr His
                   325                 330                 335
   Phe Glu Lys Leu Gly Leu Lys Ile Glu Ile Val Asp Ile Glu Glu Ile
               340                 345                 350
   Lys Asp Gly Trp Arg Ile Thr Phe Lys Arg Leu Tyr Pro Asp Ala Thr
           355                 360                 365
   Asn Glu Ile Arg Glu Leu Ile Gly Gly Lys Ala Phe Val Asp
       370                 375                 380
<210> 5
   <211> 382
   <212> PRT
   <213> Pyrococcus furiosus
<220>
   <221> misc_feature
   <222> (95)..(95)
   <223> Xaa can be any naturally occurring amino acid, except K
<400> 5
   Met Glu Asn Met Val Ser Ser Lys Phe Lys Glu Leu Leu Tyr Thr Leu
   1               5                   10                  15
   Gly Ile Pro Glu Asp Lys Val Glu Ile Leu Glu Ala Arg Gly Gly Ile
               20                  25                  30
   Met Glu Asp Glu Phe Glu Gly Ile Arg Tyr Leu Arg Phe Lys Asn Ser
           35                  40                  45
   Val Gly Lys Leu Arg Arg Gly Thr Val Leu Phe Glu Asp Gly Thr Thr
       50                  55                  60
   Val Phe Gly Phe Pro His Ile Lys Arg Ile Val Asn Leu Ser Ala Gly
   65                  70                  75                  80
   Val Arg Lys Ile Phe Lys Ser Ser Glu Phe Tyr Val Glu Glu Xaa Val
                   85                  90                  95
   Asp Gly Tyr Asn Val Arg Val Val Lys Phe Lys Asp Arg Ile Leu Gly
               100                 105                 110
   Ile Thr Arg Gly Gly Phe Ile Cys Pro Tyr Thr Thr Glu Arg Ile Ala
           115                 120                 125
   Glu Phe Val Pro Glu Glu Phe Phe Lys Asp His Lys Asp Leu Val Leu
       130                 135                 140
   Val Gly Glu Met Ala Gly Pro Glu Ser Pro Tyr Leu Val Glu Gly Pro
   145                 150                 155                 160
   Pro Tyr Val Lys Glu Asp Ile Gln Phe Phe Leu Phe Asp Ile Gln Asp
                   165                 170                 175
   Ile Lys Thr Gly Ser Ser Leu Pro Val Glu Glu Arg Leu Lys Leu Ala
               180                 185                 190
   Glu Glu Tyr Gly Ile Asn His Val Glu Val Phe Gly Arg Tyr Ser Tyr
           195                 200                 205
   Lys Asp Ile Asp Asp Leu Tyr Glu Leu Ile Glu Arg Leu Ser Arg Glu
       210                 215                 220
   Gly Arg Glu Gly Ile Val Met Lys Ser Pro Asp Met Lys Lys Ile Val
   225                 230                 235                 240
   Lys Tyr Val Thr Pro Tyr Ala Asn Ile Asn Asp Ile Lys Ile Gly Ala
                   245                 250                 255
   Arg Val Phe Tyr Glu Leu Pro Gly Gly Tyr Phe Thr Ser Arg Ile Ser
               260                 265                 270
   Arg Leu Ala Phe Tyr Ile Ala Glu Lys Lys Ile Arg Gly Glu Glu Leu
           275                 280                 285
   His Asn Leu Ala Leu Gln Leu Gly Lys Ala Leu Leu Gln Pro Leu Val
       290                 295                 300
   Glu Ala Ile His Asp Val Thr Gln Gly Asp Val Ile Ala Glu Arg Phe
   305                 310                 315                 320
   Arg Val Arg Val Arg Lys Ile Glu Thr Ala Tyr Lys Met Val Thr His
                   325                 330                 335
   Phe Glu Lys Leu Gly Leu Glu Ile Glu Ile Glu Asp Ile Glu Glu Ile
               340                 345                 350
   Glu Gly Gly Trp Arg Val Thr Phe Lys Arg Val Tyr Pro Glu Ala Thr
           355                 360                 365
   Arg Glu Ile Arg Asp Leu Ile Gly Gly Lys Ala Phe Val Asp
       370                 375                 380
<210> 6
   <211> 390
   <212> PRT
   <213> Hyperthermus butylicus
<220>
   <221> misc_feature
   <222> (100)..(100)
   <223> Xaa can be any naturally occurring amino acid, except K
<400> 6
   Met Thr Trp Ile Lys Asn Pro Glu Pro Trp Met Val Asn Leu Val Ala
   1               5                   10                  15
   Glu Lys Leu Gly Leu Asp Val Glu Arg Val Glu Thr Leu Ala Arg His
               20                  25                  30
   Gly Thr Ile Arg Phe Arg Gly Tyr Arg Asp Val Val Tyr Ala Leu Leu
           35                  40                  45
   Arg Arg Glu Ile Ala Gly His Pro Glu Gly Thr Val Val Leu Leu Glu
       50                  55                  60
   Arg Asn Gly Ala Arg Leu Val Pro Gly Tyr Pro Pro Ile Gln Arg Met
   65                  70                  75                  80
   Val Leu Pro Thr Ile Ala Leu Pro Arg His Phe Ile Asp Lys Val Val
                   85                  90                  95
   Val Glu Glu Xaa Met Asn Gly Tyr Asn Val Arg Leu Val Met Phe His
               100                 105                 110
   Arg Lys Leu Leu Ala Val Thr Arg Gly Gly Phe Ile Cys Pro Tyr Thr
           115                 120                 125
   Thr Ala Arg Leu Glu Arg Leu Ile Gly Gly Arg Val Arg Glu Leu Phe
       130                 135                 140
   Arg Glu Ile Asp Pro Glu Thr Tyr Thr Ile Ala Gly Glu Val Val Gly
   145                 150                 155                 160
   Leu Glu Asn Pro Tyr Thr Arg Tyr Phe Tyr Pro Glu Ala Pro Arg Phe
                   165                 170                 175
   Asp Tyr Phe Val Phe Asp Leu Phe His Glu Leu Lys Pro Leu Pro Pro
               180                 185                 190
   Leu Glu Arg Asn Glu Leu Leu Glu Lys Tyr Gly Leu Lys His Val Arg
           195                 200                 205
   Leu Leu Gly Val Ile Asp Lys Asn Asp Val Glu Met Phe Lys Gln Ile
       210                 215                 220
   Val Ala Glu Leu Asp Arg Glu Gly Arg Glu Gly Val Val Ala Lys Asp
   225                 230                 235                 240
   Pro Glu Tyr Arg Val Pro Pro Leu Lys Tyr Thr Thr Ser Ala Val Asn
                   245                 250                 255
   Ile Gly Asp Val Arg Tyr Gly Met Arg Phe Phe Met Glu Glu Gly Arg
               260                 265                 270
   Ser Phe Leu Phe Ser Arg Leu Leu Arg Glu Leu Phe Arg Ala Tyr Glu
           275                 280                 285
   Glu Gly Phe Gly Asp Ala Gln Leu Glu Lys Leu Ala Leu Glu Phe Gly
       290                 295                 300
   Arg Ala Ala Thr Glu Pro Ala Leu Glu Ser Ile Arg Lys Val Ala Met
   305                 310                 315                 320
   Gly Asp Met Leu Tyr Glu Glu Phe Glu Leu Val Phe Ala Asp Glu Val
                   325                 330                 335
   Glu Leu Glu Glu Phe Thr Ser Tyr Met Ala Glu Leu Gly Val Asp Ile
               340                 345                 350
   Val Val Val Ser Thr Ser Arg Glu Asp Glu Gly Leu Arg Ala Arg Met
           355                 360                 365
   Arg Lys Ile Lys Asp Thr Trp Ile Gln Leu Arg Lys Val Leu Asp Thr
       370                 375                 380
   Gly Leu Ser Pro Val Asp
   385                 390
<210> 7
   <211> 390
   <212> PRT
   <213> Artificial sequence
<220>
   <223> Synthetic peptide
<400> 7
   Met Thr Trp Ile Lys Asn Pro Glu Pro Trp Met Val Asn Leu Val Ala
   1               5                   10                  15
   Glu Lys Leu Gly Leu Asp Val Glu Arg Val Glu Thr Leu Ala Arg His
               20                  25                  30
   Gly Thr Ile Arg Phe Arg Gly Tyr Arg Asp Val Val Tyr Ala Leu Leu
           35                  40                  45
   Arg Arg Glu Ile Ala Gly His Pro Glu Gly Thr Val Val Leu Leu Glu
       50                  55                  60
   Arg Asn Gly Ala Arg Leu Val Pro Gly Tyr Pro Pro Ile Gln Arg Met
   65                  70                  75                  80
   Val Leu Pro Thr Ile Ala Leu Pro Arg His Phe Ile Asp Lys Val Val
                   85                  90                  95
   Val Glu Glu Ala Met Asn Gly Tyr Asn Val Arg Leu Val Met Phe His
               100                 105                 110
   Arg Lys Leu Leu Ala Val Thr Arg Gly Gly Phe Ile Cys Pro Tyr Thr
           115                 120                 125
   Thr Ala Arg Leu Glu Arg Leu Ile Gly Gly Arg Val Arg Glu Leu Phe
       130                 135                 140
   Arg Glu Ile Asp Pro Glu Thr Tyr Thr Ile Ala Gly Glu Val Val Gly
   145                 150                 155                 160
   Leu Glu Asn Pro Tyr Thr Arg Tyr Phe Tyr Pro Glu Ala Pro Arg Phe
                   165                 170                 175
   Asp Tyr Phe Val Phe Asp Leu Phe His Glu Leu Lys Pro Leu Pro Pro
               180                 185                 190
   Leu Glu Arg Asn Glu Leu Leu Glu Lys Tyr Gly Leu Lys His Val Arg
           195                 200                 205
   Leu Leu Gly Val Ile Asp Lys Asn Asp Val Glu Met Phe Lys Gln Ile
       210                 215                 220
   Val Ala Glu Leu Asp Arg Glu Gly Arg Glu Gly Val Val Ala Lys Asp
   225                 230                 235                 240
   Pro Glu Tyr Arg Val Pro Pro Leu Lys Tyr Thr Thr Ser Ala Val Asn
                   245                 250                 255
   Ile Gly Asp Val Arg Tyr Gly Met Arg Phe Phe Met Glu Glu Gly Arg
               260                 265                 270
   Ser Phe Leu Phe Ser Arg Leu Leu Arg Glu Leu Phe Arg Ala Tyr Glu
           275                 280                 285
   Glu Gly Phe Gly Asp Ala Gln Leu Glu Lys Leu Ala Leu Glu Phe Gly
       290                 295                 300
   Arg Ala Ala Thr Glu Pro Ala Leu Glu Ser Ile Arg Lys Val Ala Met
   305                 310                 315                 320
   Gly Asp Met Leu Tyr Glu Glu Phe Glu Leu Val Phe Ala Asp Glu Val
                   325                 330                 335
   Glu Leu Glu Glu Phe Thr Ser Tyr Met Ala Glu Leu Gly Val Asp Ile
               340                 345                 350
   Val Val Val Ser Thr Ser Arg Glu Asp Glu Gly Leu Arg Ala Arg Met
           355                 360                 365
   Arg Lys Ile Lys Asp Thr Trp Ile Gln Leu Arg Lys Val Leu Asp Thr
       370                 375                 380
   Gly Leu Ser Pro Val Asp
   385                 390
<210> 8
   <211> 385
   <212> PRT
   <213> Aeropyrum pernix
<220>
   <221> misc_feature
   <222> (99)..(99)
   <223> Xaa can be any naturally occurring amino acid, except K
<400> 8
   Met Ala Ser Ala Ala Glu Val Leu Ala Ser Ala Leu Arg Ala Val Gly
   1               5                   10                  15
   Val Asp Pro Gly Ser Val Asp Leu Glu Ala Leu Ser Thr Arg Arg Ser
               20                  25                  30
   Val Arg Val Ser Arg Phe Glu Asp Val Val Tyr Val Gly Phe Arg Arg
           35                  40                  45
   Gln Phe Arg Gly Val Pro Glu Gly Thr Leu Val Ala Phe Arg Arg Gly
       50                  55                  60
   Glu Gln Ile Val Val Trp Gly Tyr Pro Ser Ile Lys Arg Met Leu Leu
   65                  70                  75                  80
   Pro Arg Val Ala Val Pro Arg Trp Phe Pro Gly Pro Thr Val Leu Val
                   85                  90                  95
   Glu Glu Xaa Met Asn Gly Tyr Asn Val Arg Val Phe Thr Leu Gly Gly
               100                 105                 110
   Met Val Tyr Ala Ala Thr Arg Gly Gly Leu Ile Cys Pro Tyr Thr Thr
           115                 120                 125
   Arg Arg Leu Arg Arg Leu Tyr Gly Gly Ala Leu Gln Lys Ile Leu Glu
       130                 135                 140
   Asp Leu Gly Ala Glu Gly Ser Phe Ile Ala Gly Glu Val Val Gly Leu
   145                 150                 155                 160
   Glu Asn Pro Tyr Thr Arg Tyr Tyr Tyr Glu Glu Ala Pro Gly Phe Gly
                   165                 170                 175
   Tyr Phe Ile Phe Asp Ile Phe Lys Gly Gly Arg Gln Leu Pro Pro Arg
               180                 185                 190
   Val Lys Phe Ser Leu Ala Pro Glu Tyr Gly Leu Lys Thr Val Asn Leu
           195                 200                 205
   Leu Ala Glu Ile Pro Ala Thr Ala Ser Gly Val Glu Arg Leu Tyr Thr
       210                 215                 220
   Ile Val Glu Asp Leu Glu Lys Arg Gly Arg Glu Gly Val Ile Val Lys
   225                 230                 235                 240
   Asp Pro Glu Gly Arg Val Glu Pro Leu Lys Tyr Thr Thr Ser Arg Thr
                   245                 250                 255
   Asn Ile Gly Asp Ile Arg Leu Gly Met Arg Tyr Pro Phe Glu Glu Gly
               260                 265                 270
   Arg Ser Phe Leu Phe Pro Arg Ile Leu Arg Glu Ile Phe Arg Glu Trp
           275                 280                 285
   Glu Thr Gly Arg Arg Arg Tyr Gly Glu Leu Gly Glu Ala Ile Leu Ala
       290                 295                 300
   Pro Ala Ile Glu Ala Val Glu Ala Val Ser Arg Gly Gly Arg Leu Val
   305                 310                 315                 320
   Glu Glu Phe Glu Leu Val Phe Ala Asn Glu Val Glu Ala Glu Glu Val
   325 330 335
   Ile Ala Tyr Phe Ala Ser Leu Gly Val His Leu Glu Ile Ala Gly Val
   340 345 350
   Ala Arg Gly Val Asp Gly Val Arg Val Ala Phe Arg Lys Pro Arg Lys
   355 360 365
   Ser Glu Gly Glu Ile Ala Arg Ile Leu Glu Thr Gly Ile Ser Pro Leu
   370 375 380
   Asp
   385
<210> 9
   <211> 381
   <212> PRT
   <213> Staphylothermus marinus
<220>
   <221> misc_feature
   <222> (92)..(92)
   <223> Xaa can be any naturally occurring amino acid, except K
<400> 9
   Met Asp Glu Asn Glu Leu Val Asn Lys Leu Ser Asp Ala Leu Gly Ile
   1               5                   10                  15
   Glu Tyr Glu Lys Leu Ser Lys His Ile Gly Arg Ser Ile Arg Leu Met
               20                  25                  30
   Lys Tyr Gly Glu Leu Asn Tyr Val Val Phe Arg Arg Asp Leu Leu Gly
           35                  40                  45
   Tyr Arg Glu Gly Thr Thr Ile Leu Leu Gly Glu Glu Pro Leu Ile Val
       50                  55                  60
   His Gly Tyr Pro Ser Ile Gln Arg Leu Ala Phe Ile Glu Gly Val Ser
   65                  70                  75                  80
   Lys His Met Ile Asp Asn Val Val Val Glu Glu Xaa Met Asn Gly Tyr
                   85                  90                  95
   Asn Val Arg Val Val Tyr Tyr Met Asn Asn Ile Tyr Ala Ile Thr Arg
               100                 105                 110
   Gly Gly Tyr Ile Cys Pro Tyr Thr Thr Ala Arg Ile Arg Lys Leu Tyr
           115                 120                 125
   Ser Lys Asn Ile Lys Leu Ala Tyr Gln Glu Tyr Pro Asp Thr Ile Leu
       130                 135                 140
   Val Gly Glu Val Val Gly Thr Glu Asn Pro Tyr Val Ile Tyr Asp Tyr
   145                 150                 155                 160
   Pro Glu Ala Arg Gly Phe Asp Tyr Phe Ile Phe Asp Thr Met Lys Lys
                   165                 170                 175
   Asp Lys Leu Gln Pro Leu Arg Ile Arg Asp Glu Ile Ala Glu Lys Tyr
               180                 185                 190
   Ser Leu Lys Thr Val Arg Ile Leu Asp Ile Ile Asn Lys Arg Asp Ile
           195                 200                 205
   Asp Arg Leu Lys Thr Ile Ile Asn Arg Leu Glu Lys Glu Arg Arg Glu
       210                 215                 220
   Gly Val Val Leu Lys Asp Pro Tyr Gln Arg Val Pro Pro Leu Lys Tyr
   225                 230                 235                 240
   Thr Thr Ile Tyr Ile Asn Ile Arg Asp Ile Trp Glu Gly Met Arg Tyr
                   245                 250                 255
   Pro Phe Asp Glu Gly Arg Gly Tyr Leu Phe Ser Arg Ile Val Arg Leu
               260                 265                 270
   Ile Ala Gln Gly Tyr Glu Tyr Asp Trp Asn Asn Thr Glu Leu Asp Arg
           275                 280                 285
   Ile Ala Leu Lys Leu Gly Arg Ala Ile Leu Glu Pro Ala Ile Asn Ser
       290                 295                 300
   Leu Lys Lys Arg Ala Asn Gly Glu Ile Ile Ala Ser Lys Tyr Thr Leu
   305                 310                 315                 320
   Val Phe Pro Ser Glu Asp Asp Leu Ser Lys Tyr Ile Glu Tyr Ala Glu
                   325                 330                 335
   Ser Ile Gly Met Asp Phe Ile Phe Arg Val Val Glu Lys Arg Glu Asp
               340                 345                 350
   Gly Cys Ile Val Val Glu Leu Phe Lys Met Lys Glu Thr His Asn Ile
           355                 360                 365
   Tyr Thr Lys Met Leu Lys Thr Gly Tyr Ser Pro Leu Asp
       370                 375                 380
<210> 10
   <211> 390
   <212> PRT
   <213> Pyrolobus fumarii
<220>
   <221> misc_feature
   <222> (100)..(100)
   <223> Xaa can be any naturally occurring amino acid, except K
<400> 10
   Met Ile Arg Ile Pro Leu Glu Arg Trp Met Ile Glu Lys Leu Ala Glu
   1               5                   10                  15
   Ala Leu Asn Val Asn Ile Glu Glu Ala Glu Arg Leu Ala Arg Arg Arg
               20                  25                  30
   Asn Val Val Arg Leu Met Lys Trp Arg Asn Val Thr Tyr Phe Ser Leu
           35                  40                  45
   Arg Lys Asp Val Tyr Gly Leu Arg Glu Gly Thr Leu Ile Ala Val Trp
       50                  55                  60
   Pro Asp Gly Tyr Arg Val Val Pro Gly Tyr Pro Ser Ile Gln Arg Val
   65                  70                  75                  80
   Leu Leu Pro Ser Val Ala Leu Pro Lys His Phe Ile Asp Lys Ile Val
                   85                  90                  95
   Val Glu Glu Xaa Leu Asn Gly Tyr Asn Val Arg Val Val Lys Leu Arg
               100                 105                 110
   Asp Glu Ile Val Ala Val Thr Arg Gly Gly Leu Ile Cys Pro Tyr Thr
           115                 120                 125
   Thr Gln Arg Ile Arg Lys Leu Tyr Gly Asp Lys Leu Thr Ser Leu Phe
       130                 135                 140
   Arg Glu Glu Gly Glu Glu Leu Val Val Ala Gly Glu Val Ile Gly Leu
   145                 150                 155                 160
   Glu Asn Pro Tyr Val Arg Phe Tyr Tyr Pro Glu Ala Gly Gly Phe Ala
                   165                 170                 175
   Tyr Phe Ile Phe Asp Ile Val His Gly Glu Lys Phe Leu Pro Pro His
               180                 185                 190
   Glu Arg Lys Glu Ile Val Glu Lys His Gly Leu Leu His Val Pro Val
           195                 200                 205
   Leu Gly Glu Ile Asp Lys Asn Asp Ile Lys Ala Phe Arg Lys Ile Ile
       210                 215                 220
   Glu Asp Leu Glu Arg Arg Gly Arg Glu Gly Val Val Leu Lys Asp Pro
   225                 230                 235                 240
   Glu Tyr Arg Val Pro Pro Leu Lys Tyr Thr Thr Ser Phe Ile Asn Ile
                   245                 250                 255
   His Asp Ile Glu Ile Gly Met Arg Phe Pro Phe Asp Glu Gly Arg Asn
               260                 265                 270
   Tyr Leu Phe Ser Arg Ile Leu Arg Glu Ile Phe Lys Ala Val Glu Glu
           275                 280                 285
   Gly Trp Asp Asp Arg Arg Leu Leu Leu Ala Glu Gln Asn Leu Gly Lys
       290                 295                 300
   Ala Ile Leu Glu Pro Ala Ile Glu Ala Val Lys Glu Val Lys Asn Gly
   305                 310                 315                 320
   Lys Met Leu Tyr Glu Glu Phe Met Leu Pro Phe Asp Thr Arg Asp Asp
                   325                 330                 335
   Phe Glu Glu Phe Leu Asp Tyr Met Ala Ser Leu Gly Val Asp Ile Ile
               340                 345                 350
   Val Ala Gly Val Glu Gln Arg Ser Asp Gly Ser Ile Val Ala Arg Ile
           355                 360                 365
   Arg Lys Val Lys Asp Thr Trp Arg Glu Val Gln Lys Ile Leu Glu Thr
       370                 375                 380
   Gly Leu Ser Pro Ile Asp
   385                 390
<210> 11
   <211> 367
   <212> PRT
   <213> Aquifex aeolicus
<220>
   <221> misc_feature
   <222> (77)..(77)
   <223> Xaa can be any naturally occurring amino acid, except K
<400> 11
   Met Ile Ser Pro Glu Leu Val Lys Glu Ala Leu Lys Lys Lys Lys Val
   1               5                   10                  15
   Arg Ser Glu Glu Ala Phe Gly Leu Glu Tyr Leu Arg Phe Asn Asp Asp
               20                  25                  30
   Tyr Lys Asp Ile Pro Arg Gly Thr Ala Ile Phe Lys Asp Phe Ile Ile
           35                  40                  45
   Trp Gly Tyr Pro His Ile Gly Arg Ile Phe Leu Leu Glu Thr Gly Leu
       50                  55                  60
   Arg Glu Gln Phe Glu Ala Pro Phe Trp Val Glu Glu Xaa Val Asp Gly
   65                  70                  75                  80
   Tyr Asn Thr Arg Ile Phe Lys Tyr Gly Asp Asn Tyr Tyr Ala Leu Ser
                   85                  90                  95
   Arg Gly Gly Phe Ile Cys Pro Phe Thr Thr Asp Arg Leu Pro Asp Leu
               100                 105                 110
   Ile Asp Leu Arg Ile Leu Asp Glu Asn Pro Asp Leu Val Ile Cys Ala
           115                 120                 125
   Glu Val Ala Gly Pro Glu Asn Pro Tyr Ile Glu Glu Ser Pro Pro Tyr
       130                 135                 140
   Val Lys Glu Asp Val Gln Leu Phe Val Phe Asp Phe Met Lys Lys Asn
   145                 150                 155                 160
   Glu Gln Gly Phe Leu Ser Gln Glu Glu Lys Met Glu Leu Ile Glu Lys
                   165                 170                 175
   Tyr Asn Leu Pro His Val Glu Ile Leu Gly Arg Phe Thr Ala Ser Glu
               180                 185                 190
   Glu Gly Ile Lys Lys Ile Lys Glu Ile Leu Lys Arg Phe Asn Glu Glu
           195                 200                 205
   Gly Arg Glu Gly Val Val Phe Lys Glu Asp Ser Glu Arg Asn Lys Arg
       210                 215                 220
   Ala Lys Tyr Ile Thr Ser Tyr Ala Asn Leu Met Asp Ile Lys Thr Asn
   225                 230                 235                 240
   Ala Lys Asn Met Leu Gln Leu Pro Pro Glu Tyr Tyr Thr Asn Arg Ile
   245 250 255
   Leu Arg Leu Val Leu Phe Met Tyr Glu Glu Gly Leu Lys Thr Thr Glu
   260 265 270
   His Leu Tyr Glu Glu Leu Gly Arg Ala Phe Ile Asp Gly Leu Phe Gln
   275 280 285
   Ala Ile Glu Gln Phe Glu Lys Glu His Lys Val Tyr Lys Thr Phe Thr
   290 295 300
   Cys Lys Phe Arg Lys Lys Glu Asn Ala Ile Ala Leu Leu Glu Leu Leu
   305 310 315 320
   Ser Lys Thr Ser Lys His Ile Gln Val Lys Glu Arg Arg Leu Glu Lys
   325 330 335
   Glu Gly Asp Tyr Trp Arg Leu Glu Phe Asp Lys Val Phe Leu Asn Met
   340 345 350
   Thr Gly Leu Leu Gly His Leu Leu Ser Gly Gly Ile Val Tyr Asp
   355 360 365
<210> 12
   <211> 5
   <212> PRT
   <213> Artificial sequence
<220>
   <223> Synthetic peptide
<220>
   <221> MISC_FEATURE
   <222> (3)..(3)
   <223> Xaa = any amino acid
<220>
   <221> MISC_FEATURE
   <222> (4)..(4)
   <223> Xaa = D, N, or H
<400> 12
   Glu Gly Xaa Xaa Gly
   1 5
<210> 13
   <211> 5
   <212> PRT
   <213> Artificial sequence
<220>
   <223> Synthetic peptide
<220>
   <221> MISC_FEATURE
   <222> (3)..(3)
   <223> Xaa = any amino acid
<220>
   <221> MISC_FEATURE
   <222> (4)..(4)
   <223> Xaa = D, N, or H
<400> 13
   Glu Pro Xaa Xaa Gly
   1               5
<210> 14
   <211> 5
   <212> PRT
   <213> Artificial sequence
<220>
   <223> Synthetic peptide
<220>
   <221> MISC_FEATURE
   <222> (3)..(3)
   <223> Xaa= any amino acid
<220>
   <221> MISC_FEATURE
   <222> (4)..(4)
   <223> Xaa = D, N, or H
<400> 14
   Glu Ala Xaa Xaa Gly
   1               5
<210> 15
   <211> 5
   <212> PRT
   <213> Artificial sequence
<220>
   <223> Synthetic peptide
<220>
   <221> MISC_FEATURE
   <222> (3)..(3)
   <223> Xaa = any amino acid
<220>
   <221> MISC_FEATURE
   <222> (4)..(4)
   <223> Xaa = D, N, or H
<400> 15
   Glu Val Xaa Xaa Gly
   1 5
<210> 16
   <211> 5
   <212> PRT
   <213> Artificial sequence
<220>
   <223> Synthetic peptide
<220>
   <221> MISC_FEATURE
   <222> (3)..(3)
   <223> Xaa = any amino acid
<220>
   <221> MISC_FEATURE
   <222> (4)..(4)
   <223> Xaa = D, N, or H
<400> 16
   Glu Leu Xaa Xaa Gly
   1 5
<210> 17
   <211> 5
   <212> PRT
   <213> Artificial sequence
<220>
   <223> Synthetic peptide
<220>
   <221> MISC_FEATURE
   <222> (3)..(3)
   <223> Xaa = any amino acid
<220>
   <221> MISC_FEATURE
   <222> (4)..(4)
   <223> Xaa = D, N, or H
<400> 17
   Glu Ile Xaa Xaa Gly
   1 5
<210> 18
   <211> 5
   <212> PRT
   <213> Artificial sequence
<220>
   <223> Synthetic peptide
<220>
   <221> MISC_FEATURE
   <222> (3)..(3)
   <223> Xaa = any amino acid
<220>
   <221> MISC_FEATURE
   <222> (4)..(4)
   <223> Xaa = D, N, or H
<400> 18
   Glu Met Xaa Xaa Gly
   1               5
<210> 19
   <211> 5
   <212> PRT
   <213> Artificial sequence
<220>
   <223> Synthetic peptide
<220>
   <221> MISC_FEATURE
   <222> (3)..(3)
   <223> Xaa = any amino acid
<220>
   <221> MISC_FEATURE
   <222> (4)..(4)
   <223> Xaa = D, N, or H
<400> 19
   Glu Cys Xaa Xaa Gly
   1               5
<210> 20
   <211> 5
   <212> PRT
   <213> Artificial sequence
<220>
   <223> Synthetic peptide
<220>
   <221> MISC_FEATURE
   <222> (3)..(3)
   <223> Xaa = any amino acid
<220>
   <221> MISC_FEATURE
   <222> (4)..(4)
   <223> Xaa = D, N, or H
<400> 20
   Glu Phe Xaa Xaa Gly
   1 5
<210> 21
   <211> 5
   <212> PRT
   <213> Artificial sequence
<220>
   <223> Synthetic peptide
<220>
   <221> MISC_FEATURE
   <222> (3)..(3)
   <223> Xaa = any amino acid
<220>
   <221> MISC_FEATURE
   <222> (4)..(4)
   <223> Xaa = D, N, or H
<400> 21
   Glu Tyr Xaa Xaa Gly
   1 5
<210> 22
   <211> 5
   <212> PRT
   <213> Artificial sequence
<220>
   <223> Synthetic peptide
<220>
   <221> MISC_FEATURE
   <222> (3)..(3)
   <223> Xaa = any amino acid
<220>
   <221> MISC_FEATURE
   <222> (4)..(4)
   <223> Xaa = D, N, or H
<400> 22
   Glu Trp Xaa Xaa Gly
   1 5
<210> 23
   <211> 5
   <212> PRT
   <213> Artificial sequence
<220>
   <223> Synthetic peptide
<220>
   <221> MISC_FEATURE
   <222> (3)..(3)
   <223> Xaa = any amino acid
<220>
   <221> MISC_FEATURE
   <222> (4)..(4)
   <223> Xaa = D, N, or H
<400> 23
   Glu His Xaa Xaa Gly
   1               5
<210> 24
   <211> 5
   <212> PRT
   <213> Artificial sequence
<220>
   <223> Synthetic peptide
<220>
   <221> MISC_FEATURE
   <222> (3)..(3)
   <223> Xaa = any amino acid
<220>
   <221> MISC_FEATURE
   <222> (4)..(4)
   <223> Xaa = D, N, or H
<400> 24
   Glu Arg Xaa Xaa Gly
   1               5
<210> 25
   <211> 5
   <212> PRT
   <213> Artificial sequence
<220>
   <223> Synthetic peptide
<220>
   <221> MISC_FEATURE
   <222> (3)..(3)
   <223> Xaa = any amino acid
<220>
   <221> MISC_FEATURE
   <222> (4)..(4)
   <223> Xaa = D, N, or H
<400> 25
   Glu Gln Xaa Xaa Gly
   1 5
<210> 26
   <211> 5
   <212> PRT
   <213> Artificial sequence
<220>
   <223> Synthetic peptide
<220>
   <221> MISC_FEATURE
   <222> (3)..(3)
   <223> Xaa= any amino acid
<220>
   <221> MISC_FEATURE
   <222> (4)..(4)
   <223> Xaa = D, N, or H
<400> 26
   Glu Asn Xaa Xaa Gly
   1 5
<210> 27
   <211> 5
   <212> PRT
   <213> Artificial sequence
<220>
   <223> Synthetic peptide
<220>
   <221> MISC_FEATURE
   <222> (3)..(3)
   <223> Xaa = any amino acid
<220>
   <221> MISC_FEATURE
   <222> (4)..(4)
   <223> Xaa = D, N, or H
<400> 27
   Glu Glu Xaa Xaa Gly
   1 5
<210> 28
   <211> 5
   <212> PRT
   <213> Artificial sequence
<220>
   <223> Synthetic peptide
<220>
   <221> MISC_FEATURE
   <222> (3)..(3)
   <223> Xaa = any amino acid
<220>
   <221> MISC_FEATURE
   <222> (4)..(4)
   <223> Xaa = D, N, or H
<400> 28
   Glu Asp Xaa Xaa Gly
   1               5
<210> 29
   <211> 5
   <212> PRT
   <213> Artificial sequence
<220>
   <223> Synthetic peptide
<220>
   <221> MISC_FEATURE
   <222> (3)..(3)
   <223> Xaa = any amino acid
<220>
   <221> MISC_FEATURE
   <222> (4)..(4)
   <223> Xaa = D, N, or H
<400> 29
   Glu Ser Xaa Xaa Gly
   1               5
<210> 30
   <211> 5
   <212> PRT
   <213> Artificial sequence
<220>
   <223> Synthetic peptide
<220>
   <221> MISC_FEATURE
   <222> (3)..(3)
   <223> Xaa = any amino acid
<220>
   <221> MISC_FEATURE
   <222> (4)..(4)
   <223> Xaa = D, N, or H
<400> 30
   Glu Thr Xaa Xaa Gly
   1               5
<210> 31
   <211> 4
   <212> PRT
   <213> Artificial sequence
<220>
   <223> Synthetic peptide
<220>
   <221> MISC_FEATURE
   <222> (2)..(2)
   <223> Xaa = any amino acid
<220>
   <221> MISC_FEATURE
   <222> (3)..(3)
   <223> Xaa = D, N, or H
<400> 31
   Glu Xaa Xaa Gly
   1
<210> 32
   <211> 5
   <212> PRT
   <213> Artificial sequence
<220>
   <223> Synthetic peptide
<220>
   <221> MISC_FEATURE
   <222> (3)..(3)
   <223> Xaa = any amino acid
<220>
   <221> MISC_FEATURE
   <222> (4)..(4)
   <223> Xaa = D, N, or H
<400> 32
   Glu Lys Xaa Xaa Gly
   1               5

## Description

The present application claims priority to U.S. Provisional application Serial Number 62/307,658, filed March 14, 2016.

### FIELD

Provided herein are compositions, systems, and methods employing hyper-thermostable lysine-mutant ssDNA/RNA ligases that possesses both ssRNA ligase and ssDNA ligase activity. Such hyper-thermostable lysine-mutant ssDNA/RNA ligases can be used to ligate a first single-strand nucleic acid sequence with a 5' adenylated end to a second single-strand nucleic acid sequence (e.g., at a temperature of at least 75°C) to form a ligated nucleic acid sequence. The ligated nucleic acid sequence can be amplified and/or sequenced.

### BACKGROUND

DNA or RNA ligases are divalent metal ion dependent enzymes that utilize ATP or NAD+ to catalyze phosphodiester bond formation between adjacent polynucleotide termini possessing a 3'-hydroxyl and a 5'-phosphate (Tomkinson et al., PNAS, 2006; Silber et al, PNAS, 1972). Depending on their origin of species, natural occurring DNA or RNA ligases may have many unique properties, for example, substrate specificity, sequence and domain organization, optimal reaction condition such as pH, co-factor dependence, temperature and salt tolerance etc. Their activities in vitro have also been exploited in numerous molecular biology protocols, making them critical tools for modern biotechnology.

All known DNA and RNA ligases perform the catalysis via a common pathway which involves three nucleotidyl transfer reactions (Lehman et al, Science, 1974; Lindahl et al, Annu Rev Biochem, 1992). In the case of ATP-dependent DNA or RNA ligases, the first step (step 1) involves the attack on the α-phosphate of ATP by ligase, which results in release of pyrophosphate and formation of a ligase-AMP intermediate. AMP is linked covalently to the amino group of a lysine residue within a conserved sequence motif. In the second step (step 2), the AMP nucleotide is transferred to the 5'-phosphate-terminated DNA or RNA strand to form a 5'-App-DNA/RNA intermediate. In the third and final step (step 3), attack by the 3'-OH strand on the 5'-App-DNA/RNA end joins the two polynucleotides and liberates AMP.

DNA ligases catalyze the formation of phosphodiester bonds at single-stranded nicks in double-stranded DNA. In vivo, this activity is critical for maintaining genomic integrity during DNA replication, DNA recombination, and DNA excision repair. Among them, T4 DNA ligase has been widely used in vitro to join double-stranded breaks with cohesive and blunt ends. RNA ligases are typically classified into at least two broad families. The Rnl1 family includes the T4 RNA ligase 1 (Rnl1) and the tRNA ligases from fungi, yeasts, and plants. These enzymes repair breaks in single-stranded RNA. The Rnl2 family is represented by the bacteriophage T4 RNA ligase 2 (T4Rnl2) and the RNA-editing ligases from the protozoans Trypanosoma and Leishmania (Ho and Shuman, PNAS, 2002). In vivo, these enzymes primarily seal nicks in double-stranded RNA. In vitro, T4Rnl2 and its mutants have been shown to join single-stranded RNA (Yin S et al, JBC, 2003), as well as between 3'-end of single-strand RNA and 5'-end of single-strand DNA adaptor (Viollet et al, 2011, BMC Biotechnol).

It has been observed that single-strand RNA ligases can sometimes accept single-strand DNA as substrate, albeit sometimes with lower ligation efficiency. For example, T4 RNA ligase 1 (T4Rnl1) has been known for a long time and used to ligate single-strand RNA (ssRNA), and ssDNA with a lower efficiency (Lau et al., 2001). Homologs within the T4 RNA ligase 1 family exhibit single-strand DNA ligation activity. For example, thermostable single-strand DNA ligase from *Thermus scotoductus* bacteriophage TS2126, which is a T4Rnl1 homolog, can ligate ssDNA at elevated temperature around 65°C (Blondal et al, NAR, 2005) and was marketed as CircLigase by EpiCenter Biotechnologies (WO2004/027056A2). Another T4Rnl1 homolog from *Rhodothermus marinus* bacteriophage RM378 was also reported to ligate both ssRNA and ssDNA (Blondal et al, NAR, 2002). Due to its unique ssDNA ligation activity at relatively high temperature (∼65°C), CircLigase quickly finds its application in many applications, such as in the process of library preparation of high-throughput next-generation sequencing (Gansauge MT, Nat. Protol., 2013).

Advancement in next-generation sequencing (NGS) has transformed biomedical and clinical research with an accelerating momentum. One of the most crucial families of enzymes used in the NGS library preparation workflow is ligases. The significance of the ligation step is that it attaches short pieces of DNA or RNA adaptors with known sequences to the ends of the unknown library sequences, thus provide priming sites to facilitate downstream steps such as PCR or primer extension. For DNA-sequencing applications, ligation step in the NGS library preparation workflow commonly occur between double-stranded library nucleic acids fragments and double-strand adaptor, but also possible between single-stranded nucleic acids fragments and 5'-phosphorylated single-stranded adaptor. The double-stranded ligation reaction is oftentimes catalyzed by T4 DNA ligase and is usually carried out at ambient or even lower (e.g., 16°C) temperature. Single-stranded DNA ligation is currently catalyzed by enzymes such as CircLigase, with reaction temperature as high as 65°C (Gansauge MT, Nat. Protol., 2013). In other applications such as small RNA sequencing, T4Rnl1 and T4Rnl2 mutants are commonly used to attached single-stranded DNA adaptor to single-strand RNA library fragments, usually at 37°C or lower.

Zhelkovsky et al., BMC Molecular Biology, 2012, 13:24 discloses K97A and K246A mutants of the thermophilic RNA ligase of Methanobacterium thermoautotrophicum and the K97A mutation is in motif EKx(D/N/H)G and said ligase is also described in US 2014/128292 A1. In Zhelkovsky *et al.,* 2012 the ligation reaction takes place at 65°C, which is well below the claimed temperature of at least 75°C as defined in the enclosed claims, and in US 2014/128292 A1 ligation reactions are carried out at a temperature of up to 75°C. Hyper-thermostable ligases having a temperature optimum above 65° C are known, for example, from Nakatani et al., Journal of Bacteriology, Nov. 2000, p. 6424-6433 and Keppetipola and Shuman, Journal of Bacteriology, Oct. 2000, p. 6902-6908.

### SUMMARY

The invention is set out in the appended claims. The examples of the description which do not fall within the scope of the claims are provided for illustrative purposes only and do not form part of the invention. Provided herein are compositions, systems, and methods employing hyper-thermostable lysine-mutant ssDNA/RNA ligases that possesses both ssRNA ligase and ssDNA ligase activity. Such hyper-thermostable lysine-mutant ssDNA/RNA ligases can be used to ligate an first single stranded nucleic acid sequence with a 5' adenylated end to a second single stranded nucleic acid sequence (e.g., at a temperature of at least 75°C) to form a ligated nucleic acid sequence. The ligated nucleic acid sequence can be amplified and/or sequenced.

Provided herein are methods of ligating single-stranded nucleic acid comprising: a) combining in a reaction mixture: i) a hyper-thermostable lysine-mutant ssDNA/RNA ligase which is a mutated version of a precursor hyper-thermostable ssRNA ligase (e.g., a precursor ssRNA ligase shown in Table 2, or truncated or mutated versions thereof), wherein the precursor hyper-thermostable ssRNA ligase has a Motif I EKx(D/N/H)G (SEQ ID NO:32) and possess ssRNA ligase activity, but not ssDNA ligase activity, at a temperature of at least 75°C (e.g., at least 75 ... 85 ... 95 ... or 100 °C, or between 75-100 °C), and wherein the hyper-thermostable lysine-mutant ssDNA/RNA ligase has an amino acid substitution at the K (lysine) in the Motif I, and possesses both ssRNA ligase and ssDNA ligase activity at a temperature of at least 75°C (e.g., at least 75 ... 85 ... 95 ... or 100 °C, or between 75-100 °C), ii) a first single stranded nucleic acid sequence with a 5' end base that is adenylated (or becomes adenylated while in the reaction mixture), and iii) a second single stranded nucleic acid sequence with a 3' end base; and b) incubating the reaction mixture at a temperature of at least 75°C (e.g., at least 75 ... 85 ... 95 ... or 100 °C, or between 75-100 °C) such that the hyper-thermostable lysine-mutant ssDNA/RNA ligase ligates the 5' adenylated end of the first single stranded nucleic acid sequence to the 3' end of the second single stranded nucleic acid sequence to form a ligated nucleic acid sequence. The incubating the reaction mixture may be at a temperature of at least 85°C or at least 95°C. The hyper-thermostable lysine-mutant ssDNA/RNA ligase may be an adenylation-deficient ATP-dependent ligase (i.e., a ligase that cannot form the AMP-ligase intermediate by reacting with ATP).

Provided herein are systems and kits comprising: a) a hyper-thermostable lysine-mutant ssDNA/RNA ligase which is a mutated version of a precursor hyper-thermostable ssRNA ligase (e.g., a precursor ssRNA ligase shown in Table 2, or truncated or mutated versions thereof), wherein the precursor hyper-thermostable ssRNA ligase has a Motif I EKx(D/N/H)G (SEQ ID NO:32) and possess ssRNA ligase activity, but not ssDNA ligase activity, at a temperature of at least 75°C, and wherein the hyper-thermostable lysine-mutant ssDNA/RNA ligase has an amino acid substitution at the K (lysine) in the Motif I, and possesses both ssRNA ligase and ssDNA ligase activity at a temperature of at least 75°C, b) a first single stranded nucleic acid sequence with a 5' end base that is adenylated; and c) a second single stranded nucleic acid sequence with a 3' end base. The kits and systems may further comprise a first container and a second container, and wherein the hyper-thermostable lysine-mutant ssDNA/RNA ligase is in the first container, and the first and/or second single-stranded nucleic acid sequence is in the second container.

Provided herein are compositions comprising: a hyper-thermostable lysine-mutant ssDNA/RNA ligase which is a mutated version of a precursor hyper-thermostable ssRNA ligase (e.g., a precursor ssRNA ligase shown in Table 2 or truncated or mutated versions thereof), wherein the precursor hyper-thermostable ssRNA ligase has a Motif I EKx(D/N/H)G (SEQ ID NO:32) and possess ssRNA ligase activity, but not ssDNA ligase activity, at a temperature of at least 75°C (e.g., at a temperature between 75-85 or 75-100 °C), and wherein the hyper-thermostable lysine-mutant ssDNA/RNA ligase has an amino acid substitution at the K (lysine) in the Motif I, and possesses both ssRNA ligase and ssDNA ligase activity at a temperature of at least 75°C (e.g., at a temperature between 75-85 or 75-100 °C). The compositions may further comprise at least one of the following: a) a first single stranded nucleic acid sequence with a 5' end base that is adenylated, and b) a second single stranded nucleic acid sequence with a 3' end base.

The 5' end base of the first single stranded nucleic acid sequence may be a DNA base, and wherein the 3' end base of the second single stranded nucleic acid sequence may be an RNA base or a DNA base. The 5' end base of the first single stranded nucleic acid sequence may be a DNA base or an RNA base, and wherein the 3' end base of the second single stranded nucleic acid sequence may be a DNA base. All or nearly all of the bases in the first single stranded nucleic acid sequence may be DNA bases. All of the bases, or nearly all of the bases, in the second single stranded nucleic acid sequence may be DNA bases.

The amino acid substitution for the K (lysine) is an amino acid selected from the group consisting of: serine (S), cysteine (C), valine (V), threonine (T), and Glycine (G) as defined in the claims. The precursor hyper-thermostable ssRNA ligase may be a wild-type hyper-thermostable ssRNA ligase. The wild-type hyper-thermostable ssRNA ligase may be from a species selected from the group consisting of: *Thermococcus kodakarensis, Pyrococcus, yayanosii, Pyrococcus horikoshii, Pyrococcus abyssi, Pyrococcus furiosus, Hyperthermus butylicus, Aeropyrum pernix, Staphylothermus marinus, Pyrolobus fumarii, and Aquifex aeolicus.* The hyper-thermostable lysine-mutant ssDNA/RNA ligase may be encoded by one of the amino acid sequences shown in SEQ ID NO:1-11. The hyper-thermostable lysine-mutant ssDNA/RNA ligase may be encoded by an amino acid sequence that has at least 85%, 95%, or 99% sequence identity to one of the amino acid sequences shown in SEQ ID NO:1-11 (e.g., at least 85% ... 90% ... 95% ... 98% ... 99% ... 99.5%). The hyper-thermostable lysine-mutant ssDNA/RNA ligase may be encoded by an N-terminal or C-terminal truncated amino acid sequence shown in SEQ ID NO:1-11 (e.g., where the SEQ ID NO is missing 1-40 amino acids at either the N or C terminal, yet still has the same ligase activity as the full length sequence).

The hyper-thermostable lysine-mutant ssDNA/RNA ligase may comprise a Motif sequence selected from the group consisting of: EGx(D/N/H)G (SEQ ID NO:12), EPx(D/N/H)G (SEQ ID NO:13), EAx(D/N/H)G (SEQ ID NO:14), EVx(D/N/H)G (SEQ ID NO:15), ELx(D/N/H)G (SEQ ID NO:16), E Ix(D/N/H)G (SEQ ID NO:17), EMx(D/N/H)G (SEQ ID NO:18), ECx(D/N/H)G (SEQ ID NO:19), EFx(D/N/H)G (SEQ ID NO:20), EYx(D/N/H)G (SEQ ID NO:21), EWx(D/N/H)G (SEQ ID NO:22), EHx(D/N/H)G (SEQ ID NO:23), ERx(D/N/H)G (SEQ ID NO:24), EQx(D/N/H)G (SEQ ID NO:25), ENx(D/N/H)G (SEQ ID NO:26), EEx(D/N/H)G (SEQ ID NO:27), EDx(D/N/H)G (SEQ ID NO:28), ESx(D/N/H)G (SEQ ID NO:29), ETx(D/N/H)G (SEQ ID NO:30) and Ex(D/N/H)G (SEQ ID NO:31), point deletion of lysine); wherein x is any amino acid. x may be an amino acid with a small or smaller side chain compared to other amino acids.

The first single stranded nucleic acid sequence may be a sequencing adapter (e.g., a next generation sequence adapter). The second single stranded nucleic acid sequence may comprise a sequencing library fragment. The methods may further comprising: c) sequencing the ligated nucleic acid sequence.

Provided herein are methods comprising: a) combining in a reaction mixture: i) a single-stranded RNA ligase from *Pyrococcus furiousus* (PfuRn12, WP_014835129.1) (or truncated or mutated version thereof), ii) a single-stranded nucleic acid sequence with an un-adenylated 5' end, and iii) ATP molecules; and b) incubating the reaction mixture under condition such that the PfuRnl2 adenylates the un-adenylated 5' end of the single-stranded nucleic acid sequence, thereby generating a first single stranded nucleic acid sequence with a 5' end base that is adenylated. The incubating may be at a temperature of at least 75°C (e.g., at least 75 ... 85 ... 95 ... or 100 °C; or between 75-85 or between 75-100 °C). The combining in the reaction mixture may further include: iv) a second single stranded nucleic acid sequence with a 3' end base, v) a hyper-thermostable lysine-mutant ssDNA/RNA ligase which is a mutated version of a precursor hyper-thermostable ssRNA ligase, wherein the precursor hyper-thermostable ssRNA ligase has a Motif I EKx(D/N/H)G (SEQ ID NO:32) and possess ssRNA ligase activity, but not ssDNA ligase activity, at a temperature of at least 75°C, and wherein the hyper-thermostable lysine-mutant ssDNA/RNA ligase has an amino acid substitution at the K (lysine) in the Motif I, and possesses both ssRNA ligase and ssDNA ligase activity at a temperature of at least 75°C. The hyper-thermostable lysine-mutant ssDNA/RNA ligase may ligate the 5' adenylated end of the first single stranded nucleic acid sequence to the 3' end of the second single stranded nucleic acid sequence to form a ligated nucleic acid sequence.

Provided herein are compositions, kits, or systems comprising: a) a single-stranded RNA ligase from *Pyrococcus furiousus* (PfuRn12, WP_014835129.1) (or truncated or mutated version thereof with the same or similar activity), and b) a hyper-thermostable lysine-mutant ssDNA/RNA ligase which is a mutated version of a precursor hyper-thermostable ssRNA ligase, wherein the precursor hyper-thermostable ssRNA ligase has a Motif I EKx(D/N/H)G (SEQ ID NO:32) and possess ssRNA ligase activity, but not ssDNA ligase activity, at a temperature of at least 75°C, and wherein the hyper-thermostable lysine-mutant ssDNA/RNA ligase has an amino acid substitution at the K (lysine) in the Motif I, and possesses both ssRNA ligase and ssDNA ligase activity at a temperature of at least 75°C. In other embodiments, the compositions, kits, and systems further comprise: c) a single-stranded nucleic acid sequence with an un-adenylated 5' end.

### BRIEF DESCRIPTION OF THE DRAWINGS

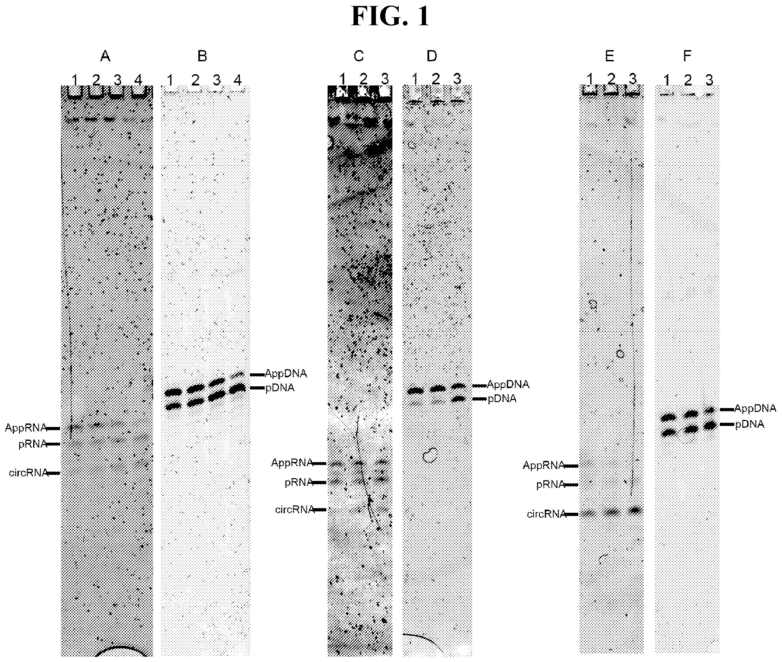

Figure 1 shows activities of wild-type PhoRnl2 (A and B), PfuRnl2 (C and D), HbuRnl2 (E and F) on 5'-phosphorylated single-stranded (ss) RNA and DNA in varying concentration of ATP. (A) Activity of PhoRnl2 on 5'-phosphorylated ssRNA. Lane 1, 100uM ATP; lane 2, 10uM ATP; lane 3, 1uM ATP; lane 4, no ATP added. pRNA, 5'-phosphorylated ssRNA; AppRNA, 5'-adenylated ssRNA; circRNA, circular ssRNA. (B) Activity of PhoRnl2 on 5'-phosphorylated ssDNA. Lane 1, 100uM ATP; lane 2, 10uM ATP; lane 3, 1uM ATP; lane 4, no ATP added. pDNA, 5'-phosphorylated ssDNA; AppDNA, 5'-adenylated ssDNA. (C) Activity of PfuRnl2 on 5'-phosphorylated ssRNA. Lane 1, 100uM ATP; lane 2, 10uM ATP; lane 3, 1uM ATP. (D) Activity of PfuRnl2 on 5'-phosphorylated ssDNA. Lane 1, 100uM ATP; lane 2, 10uM ATP; lane 3, 1uM ATP. (E) Activity of HbuRnl2 on 5'-phosphorylated ssRNA. Lane 1, 100uM ATP; lane 2, 10uM ATP; lane 3, 1uM ATP. (F) Activity of HbuRnl2 on 5'-phosphorylated ssDNA. Lane 1, 100uM ATP; lane 2, 10uM ATP; lane 3, 1uM ATP. All reactions were incubated at 90°C for 1 hour and resolved on 20% polyacrylamide denaturing gel containing 7M urea. Gels were stained using Sybr Gold and visualized using GE ImageQuant LAS scanner.

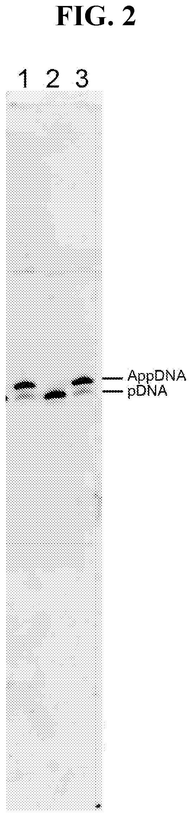

Figure 2 shows activity of wild-type HbuRnl2 on 5'-adenylated ssDNA with and without ATP. Lane 1, 5'-adenylated ssDNA with purified wild-type HbuRnl2 and 100uM ATP; lane 2, 5'-adenylated ssDNA with purified wild-type HbuRnl2 and no ATP; lane 3, 5'-adenylated ssDNA only. All reactions were incubated at 90°C for 1 hour and resolved on 20% polyacrylamide denaturing gel containing 7M urea. Gels were stained using Sybr Gold and visualized using GE ImageQuant LAS scanner.

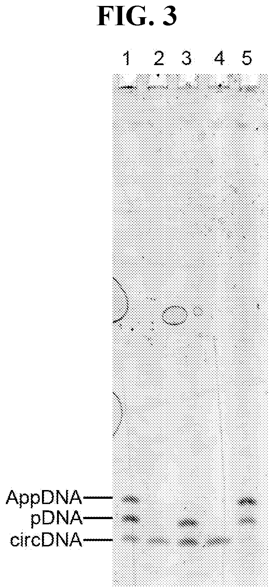

Figure 3 shows the activity of mutant HbuRn12K106A on 5'-adenylated ssDNA. Phosphorylated ssDNA was first adenylated using 5'-adenylation kit (NEB#E2610) and the product was purified from reaction (shown in Lane 5). In a 10ul total reaction volume, reaction buffer contains 70mM Tris-HCl (pH=7.6), 10mM MgCl₂, 5mM DTT. Lane 1, 1ul 5'-adenylated ssDNA with 1ul HbuRnl2K106A, incubated at 90°C for 1 hour. Lane 2, 1ul 5'-adenylated ssDNA with 1ul HbuRnl2K106A, incubated at 90°C for 1 hour, after which 1ul Exonuclease I (NEB#M0293) was added and further incubated at 37°C for 1 hour. Lane 3, 1ul 5'-adenylated ssDNA with 1ul MthRnl (NEB#E2610), incubated at 65°C for 1 hour. Lane 4, 1ul 5'-adenylated ssDNA with 1ul MthRnl (NEB#E2610), incubated at 65°C for 1 hour, after which 1ul Exonuclease I (NEB#M0293) was added and further incubated at 37°C for 1 hour. All reactions were run on 20% polyacrylamide denaturing gel containing 7M urea.

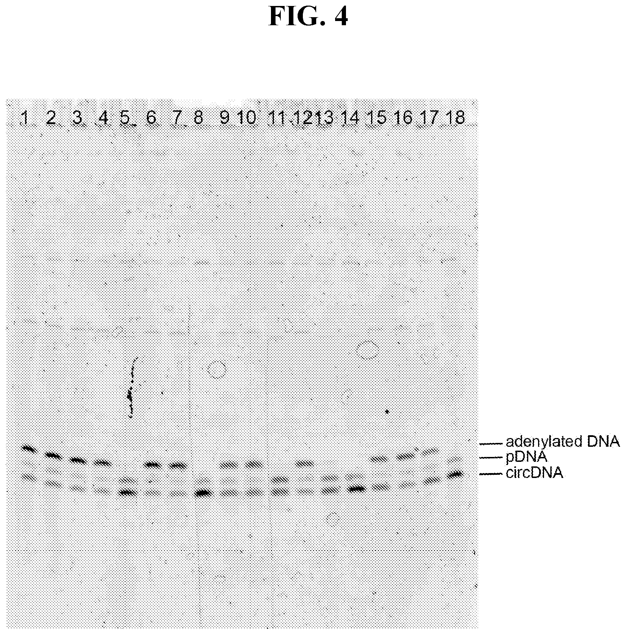

Figure 4 shows the activity of the HbuRnl2 lysine 106 mutants. All mutants were His-tagged and purified using Ni-NTA beads. Purified mutants were assayed for their ssDNA ligation activity using 5'-adenylated ssDNA. 1ul purified HbuRnl2K106A mutant was added to a 10ul ligation containing 70mM Tris-HCl, 5mM DTT, 5mM MnCl₂ (pH 7.6@25°C) and 10 pmol of adenylated ssDNA substrate. The reactions were incubated at 90°C for 1 hour and resolved on 20% denaturing polyacrylamide gel with 7M urea. The gel was stained using SYBR Gold and scanned using GE ImageQuant LAS scanner. Circularized ssDNA band was labeled at the right of the gel. Disappearance of the adenylated band and the accumulation of the circularized band suggest the activity of the mutant. Lanes from 1 to 18 correspond to mutants: 1=K106L; 2=K106I; 3=K106W; 4=K106M; 5=K106T; 6=K106X (point deletion); 7=K106H; 8=K106G; 9=K106N; 10=K106Y; 11=K106V; 12=K106P; 13=K106C; 14=K106S; 15=K106E; 16=K106F; 17=K106R; 18=K106A.

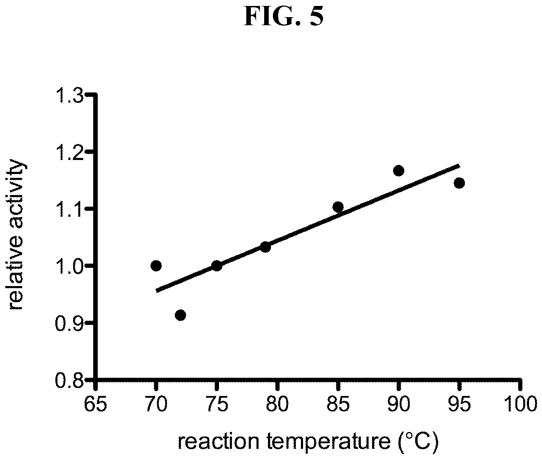

Figure 5 shows the reaction temperature profile of the mutant HbuRnl2K106A. Ligation using 5'-adenylated ssDNA were conducted in Bio-rad MyCycler using temperature gradient option. All reactions were run on 20% polyacrylamide denaturing gel containing 7M urea. The gel was stained using Sybr Gold and scanned using GE Imager LAS. The gel image was analyzed by ImageJ. The circularized ssDNA band was converted to numerical values, normalized by the value at 70°C, and plotted against reaction temperature (X-axis). The data points were fitted by using a linear curve fitting.

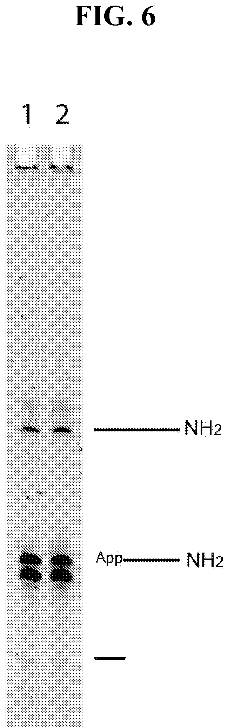

Figure 6 shows the inter-molecule ligation between two pieces of ssDNA using HbuRnl2K106A. Ligation reactions were done in 10ul volume, with buffer containing 70mM Tris-HCl (pH=7.6), 10mM MgCl₂, 5mM DTT, 5mM Mn²⁺. Each reaction contains 2pmol short ssDNA (lowest band) and about 20pmol 5'-adenylated 3'-NH2 blocked adaptor (top of the middle two bands, the lower band is phosphorylated adaptor, partly due to incomplete adenylation). In addition, lane 1 contains 12% PEG8000 and lane 2 contains 15% PEG8000. Both reactions were incubated at 90°C for 1 hour and resolved on 20% polyacrylamide denaturing gel containing 7M urea. Gels were stained using Sybr Gold and visualized using GE ImageQuant LAS scanner.

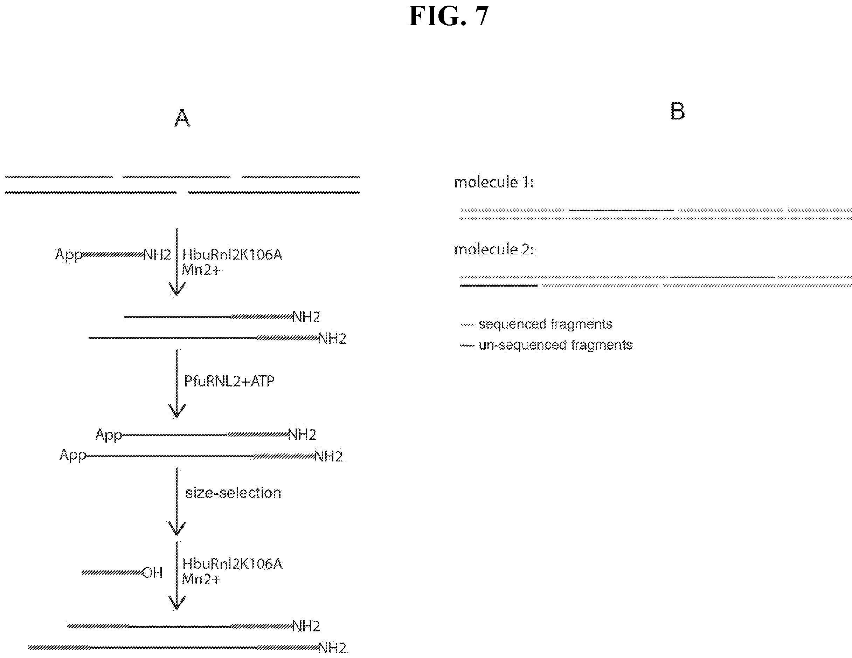

Figure 7 shows exemplary ssDNA ligation based NGS library preparation.

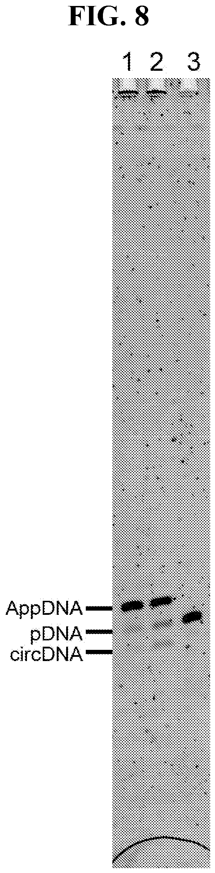

Figure 8 shows the use of PfuRnl2 and HbuRn12K106A as a mixture. All 10ul reactions were done in 70mM Tris-HCl (pH=7.6), 10mM MgCl₂, 5mM DTT. Reactions were incubated at 90C for 1 hour and resolved on 20% polyacrylamide denaturing gel containing 7M urea. Gels were stained using Sybr Gold and visualized using GE ImageQuant LAS scanner. Lane 3, 5'-phosphorylated DNA only; lane 1, 5'-phosphorylated DNA with 1ul PfuRnl2 and 1ul HbuRn12K106A and 100uM ATP; lane 2, 5'-phosphorylated DNA with 1ul PfuRnl2 and 1ul HbuRn12K106A and 10uM ATP.

### DEFINITIONS

In this application, the 5'-adenylated end of ssDNA or ssRNA if referred to as "donor" the end, and the 3'-OH end of ssDNA or ssRNA is referred to as the "acceptor" end.

### DETAILED DESCRIPTION

Provided herein are compositions, systems, and methods employing hyper-thermostable lysine-mutant ssDNA/RNA ligases that possess both ssRNA ligase and ssDNA ligase activity. Such hyper-thermostable lysine-mutant ssDNA/RNA ligases may be used to ligate a first single stranded nucleic acid sequence with a 5' adenylated end to a second single stranded nucleic acid sequence (e.g., at a temperature of at least 75°C) to form a ligated nucleic acid sequence. The ligated nucleic acid sequence may be amplified and/or sequenced.

The hyper-thermostable lysine-mutants may be as provided in SEQ ID Nos:1-11 in Table 1 below, or N or C terminal truncated, versions thereof.

**TABLE 1**

| **Hyper-thermostable Lysine-Mutant ssDNA/RNA Ligases** | | |
|---|---|---|
| Amino Acid Sequence | SEQ ID NO: | Species name |
| | 1 | *Thermococcus kodakarensis* |
| Where X is any amino acid except K. | | |
| | 2 | *Pyrococcus yayanosii* |
| Where X is any amino acid except K. | | |
| | 3 | *Pyrococcus horikoshii* |
| | | |
| Where X is any amino acid except K. | | |
| | 4 | *Pyrococcus abyssi* |
| Where X is any amino acid except K. | | |
| | 5 | *Pyrococcus furiosus* |
| Where X is any amino acid except K. | | |
| | 6 | *Hyperthermus butylicus* |
| Where X is any amino acid except K. | | |
| | 7 | *HbuRN12K106A* |
| | 8 | *Aeropyrum pernix* |
| Where X is any amino acid except K. | | |
| | 9 | *Staphylothermus marinus* |
| Where X is any amino acid except K. | | |
| | 10 | *Pyrolobus fumarii* |
| Where X is any amino acid except K. | | |
| | 11 | *Aquifex aeolicus* |
| Where X is any amino acid except K. | | |

The sequences in Table 1 above or Table 2 below may be used to perform a sequence search (e.g., using BLAST) to find other hyper-thermostable ssRNA ligases from other species (e.g., by finding those with 30% ... 50% ... 60% or more homology). Once such ligases are identified, Motif I in such sequences can be located, and the lysine (K) in such Motif I can be mutated to another amino acid, preferably an alanine (A), serine (S), cysteine (C), valine (V), threonine (T), and Glycine (G). Such candidate enzymes can then be screened for ssDNA and ssRNA activities using (and thermostability), for example, using the same procedure as in Example 1 below. In this regard finding and designing hyper-thermostable lysine-mutant ssDNA/RNA ligases may be readily accomplished.

The hyper-thermostable lysine-mutant ssDNA/RNA ligases may be used in sequencing methods, such as in attaching adapters to library fragments for subsequent sequencing. For example, the disclosure provided herein may find use in a Second Generation (a.k.a. Next Generation or Next-Gen), Third Generation (a.k.a. Next-Next-Gen), or Fourth Generation (a.k.a. N3-Gen) sequencing technology including, but not limited to, pyrosequencing, sequencing-by-ligation, single molecule sequencing, sequence-by-synthesis (SBS), semiconductor sequencing, massive parallel clonal, massive parallel single molecule SBS, massive parallel single molecule real-time, massive parallel single molecule real-time nanopore technology, etc. Morozova and Marra provide a review of some such technologies in Genomics, 92: 255 (2008).

Any number of DNA sequencing techniques are suitable, including fluorescence-based sequencing methodologies (See, e.g., Birren et al., Genome Analysis: Analyzing DNA, 1, Cold Spring Harbor, N.Y.). The present disclosure may find use in automated sequencing techniques understood in that art. The present technology may find use in parallel sequencing of partitioned amplicons (PCT Publication No: WO2006084132). The technology may find use in DNA sequencing by parallel oligonucleotide extension (See, e.g., U.S. Pat. No. 5,750,341, and U.S. Pat. No. 6,306,597). Additional examples of sequencing techniques in which the technology finds use include the Church polony technology (Mitra et al., 2003, Analytical Biochemistry 320, 55-65; Shendure et al., 2005 Science 309, 1728-1732; U.S. Pat. No. 6,432,360, U.S. Pat. No. 6,485,944, U.S. Pat. No. 6,511,803), the 454 picotiter pyrosequencing technology (Margulies et al., 2005 Nature 437, 376-380; US 20050130173), the Solexa single base addition technology (Bennett et al., 2005, Pharmacogenomics, 6, 373-382; U.S. Pat. No. 6,787,308; U.S. Pat. No. 6,833,246), the Lynx massively parallel signature sequencing technology (Brenner et al. (2000). Nat. Biotechnol. 18:630-634; U.S. Pat. No. 5,695,934; U.S. Pat. No. 5,714,330), and the Adessi PCR colony technology (Adessi et al. (2000). Nucleic Acid Res. 28, E87; WO 00018957).

Next-generation sequencing (NGS) methods share the common feature of massively parallel, high-throughput strategies, with the goal of lower costs in comparison to older sequencing methods (see, e.g., Voelkerding et al., Clinical Chem., 55: 641-658, 2009; MacLean et al., Nature Rev. Microbiol., 7: 287-296). NGS methods can be broadly divided into those that typically use template amplification and those that do not. Amplification-requiring methods include pyrosequencing commercialized by Roche as the 454 technology platforms (e.g., GS 20 and GS FLX), Life Technologies/Ion Torrent, the Solexa platform commercialized by Illumina, GnuBio, and the Supported Oligonucleotide Ligation and Detection (SOLiD) platform commercialized by Applied Biosystems. Non-amplification approaches, also known as single-molecule sequencing, are exemplified by the HeliScope platform commercialized by Helicos BioSciences, and emerging platforms commercialized by VisiGen, Oxford Nanopore Technologies Ltd., and Pacific Biosciences, respectively.

In pyrosequencing (Voelkerding et al., Clinical Chem., 55: 641-658, 2009; MacLean et al., Nature Rev. Microbiol., 7: 287-296; U.S. Pat. No. 6,210,891; U.S. Pat. No. 6,258,568), template DNA is fragmented, end-repaired, ligated to adapters, and clonally amplified in-situ by capturing single template molecules with beads bearing oligonucleotides complementary to the adaptors. Each bead bearing a single template type is compartmentalized into a water-in-oil microvesicle, and the template is clonally amplified using a technique referred to as emulsion PCR. The emulsion is disrupted after amplification and beads are deposited into individual wells of a picotitre plate functioning as a flow cell during the sequencing reactions. Ordered, iterative introduction of each of the four dNTP reagents occurs in the flow cell in the presence of sequencing enzymes and luminescent reporter such as luciferase. In the event that an appropriate dNTP is added to the 3' end of the sequencing primer, the resulting production of ATP causes a burst of luminescence within the well, which is recorded using a CCD camera. It is possible to achieve read lengths greater than or equal to 400 bases, and 10⁶ sequence reads can be achieved, resulting in up to 500 million base pairs (Mb) of sequence.

In the Solexa/Illumina platform (Voelkerding et al., Clinical Chem., 55: 641-658, 2009; MacLean et al., Nature Rev. Microbiol., 7: 287-296; U.S. Pat. No. 6,833,246; U.S. Pat. No. 7,115,400; U.S. Pat. No. 6,969,488), sequencing data are produced in the form of shorter-length reads. In this method, single-stranded fragmented DNA is end-repaired to generate 5'-phosphorylated blunt ends, followed by Klenow-mediated addition of a single A base to the 3' end of the fragments. A-addition facilitates addition of T-overhang adaptor oligonucleotides, which are subsequently used to capture the template-adaptor molecules on the surface of a flow cell that is studded with oligonucleotide anchors. The anchor is used as a PCR primer, but because of the length of the template and its proximity to other nearby anchor oligonucleotides, extension by PCR results in the "arching over" of the molecule to hybridize with an adjacent anchor oligonucleotide to form a bridge structure on the surface of the flow cell. These loops of DNA are denatured and cleaved. Forward strands are then sequenced with reversible dye terminators. The sequence of incorporated nucleotides is determined by detection of post-incorporation fluorescence, with each fluor and block removed prior to the next cycle of dNTP addition. Sequence read length ranges from 36 nucleotides to over 250 nucleotides, with overall output exceeding 1 billion nucleotide pairs per analytical run.

Sequencing nucleic acid molecules using SOLiD technology (Voelkerding et al., Clinical Chem., 55: 641-658, 2009; MacLean et al., Nature Rev. Microbiol., 7: 287-296; U.S. Pat. No. 5,912,148; U.S. Pat. No. 6,130,073) also involves fragmentation of the template, ligation to oligonucleotide adapters, attachment to beads, and clonal amplification by emulsion PCR. Following this, beads bearing template are immobilized on a derivatized surface of a glass flow-cell, and a primer complementary to the adaptor oligonucleotide is annealed. However, rather than utilizing this primer for 3' extension, it is instead used to provide a 5' phosphate group for ligation to interrogation probes containing two probe-specific bases followed by 6 degenerate bases and one of four fluorescent labels. In the SOLiD system, interrogation probes have 16 possible combinations of the two bases at the 3' end of each probe, and one of four fluors at the 5' end. Fluor color, and thus identity of each probe, corresponds to specific color-space coding schemes. Multiple rounds (usually 7) of probe annealing, ligation, and fluor detection are followed by denaturation, and then a second round of sequencing using a primer that is offset by one base relative to the initial primer. In this manner, the template sequence can be computationally re-constructed, and template bases are interrogated twice, resulting in increased accuracy. Sequence read length averages 35 nucleotides, and overall output exceeds 4 billion bases per sequencing run.

The technology described herein may find use in nanopore sequencing (see, e.g., Astier et al., J. Am. Chem. Soc. 2006 Feb 8; 128(5):1705-10). The theory behind nanopore sequencing has to do with what occurs when a nanopore is immersed in a conducting fluid and a potential (voltage) is applied across it. Under these conditions a slight electric current due to conduction of ions through the nanopore can be observed, and the amount of current is exceedingly sensitive to the size of the nanopore. As each base of a nucleic acid passes through the nanopore, this causes a change in the magnitude of the current through the nanopore that is distinct for each of the four bases, thereby allowing the sequence of the DNA molecule to be determined.

The technology described herein may find use in HeliScope by Helicos BioSciences (Voelkerding et al., Clinical Chem., 55: 641-658, 2009; MacLean et al., Nature Rev. Microbiol., 7: 287-296; U.S. Pat. No. 7,169,560; U.S. Pat. No. 7,282,337; U.S. Pat. No. 7,482,120; U.S. Pat. No. 7,501,245; U.S. Pat. No. 6,818,395; U.S. Pat. No. 6,911,345; U.S. Pat. No. 7,501,245). Template DNA is fragmented and polyadenylated at the 3' end, with the final adenosine bearing a fluorescent label. Denatured polyadenylated template fragments are ligated to poly(dT) oligonucleotides on the surface of a flow cell. Initial physical locations of captured template molecules are recorded by a CCD camera, and then label is cleaved and washed away. Sequencing is achieved by addition of polymerase and serial addition of fluorescently-labeled dNTP reagents. Incorporation events result in fluor signal corresponding to the dNTP, and signal is captured by a CCD camera before each round of dNTP addition. Sequence read length ranges from 25-50 nucleotides, with overall output exceeding 1 billion nucleotide pairs per analytical run.

The Ion Torrent technology is a method of DNA sequencing based on the detection of hydrogen ions that are released during the polymerization of DNA (see, e.g., Science 327(5970): 1190 (2010); U.S. Pat. Appl. Pub. Nos. 20090026082, 20090127589, 20100301398, 20100197507, 20100188073, and 20100137143). A microwell contains a template DNA strand to be sequenced. Beneath the layer of microwells is a hypersensitive ISFET ion sensor. All layers are contained within a CMOS semiconductor chip, similar to that used in the electronics industry. When a dNTP is incorporated into the growing complementary strand a hydrogen ion is released, which triggers a hypersensitive ion sensor. If homopolymer repeats are present in the template sequence, multiple dNTP molecules will be incorporated in a single cycle. This leads to a corresponding number of released hydrogens and a proportionally higher electronic signal. This technology differs from other sequencing technologies in that no modified nucleotides or optics is used. The per-base accuracy of the Ion Torrent sequencer is ∼99.6% for 50 base reads, with ∼100 Mb to 100Gb generated per run. The read-length is 100-300 base pairs. The accuracy for homopolymer repeats of 5 repeats in length is ∼98%. The benefits of ion semiconductor sequencing are rapid sequencing speed and low upfront and operating costs.

The technology disclosed herein finds use in another nucleic acid sequencing approach developed by Stratos Genomics, Inc. and involves the use of Xpandomers. This sequencing process typically includes providing a daughter strand produced by a template-directed synthesis. The daughter strand generally includes a plurality of subunits coupled in a sequence corresponding to a contiguous nucleotide sequence of all or a portion of a target nucleic acid in which the individual subunits comprise a tether, at least one probe or nucleobase residue, and at least one selectively cleavable bond. The selectively cleavable bond(s) is/are cleaved to yield an Xpandomer of a length longer than the plurality of the subunits of the daughter strand. The Xpandomer typically includes the tethers and reporter elements for parsing genetic information in a sequence corresponding to the contiguous nucleotide sequence of all or a portion of the target nucleic acid. Reporter elements of the Xpandomer are then detected. Additional details relating to Xpandomer-based approaches are described in, for example, U.S. Pat. Pub No. 20090035777.

Other single molecule sequencing methods include real-time sequencing by synthesis using a VisiGen platform (Voelkerding et al., Clinical Chem., 55: 641-58, 2009; U.S. Pat. No. 7,329,492; U.S. Pat. App. Ser. No. 11/671956; U.S. Pat. App. Ser. No. 11/781166) in which immobilized, primed DNA template is subjected to strand extension using a fluorescently-modified polymerase and florescent acceptor molecules, resulting in detectible fluorescence resonance energy transfer (FRET) upon nucleotide addition.

Provided herein are compositions, kits, and systems comprising a hyper-thermostable lysine-mutant ssDNA/RNA ligase encoded by SEQ ID NOs:1-11, or encoded by a sequence with substantial identity with SEQ ID NOs:1-11. As applied to such polypeptides, the term "substantial identity" means that two peptide sequences, when optimally aligned, such as by the programs GAP or BESTFIT using default gap weights, share at least 80 percent sequence identity, or at least 90 percent sequence identity, or at least 95 percent sequence identity or more (e.g., 95% ... 97% ... or 99% percent sequence identity). Residue positions which are not identical may differ by conservative amino acid substitutions. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. The conservative amino acids substitution groups may be: valine-leucine-isoleucine, phenylalanine-tyrosine, lysine-arginine, alanine-valine, and asparagine-glutamine. Provided herein are peptides that have substantial identity to at least a portion of the amino acid sequences shown in SEQ ID NOS: 1-11.

### EXPERIMENTAL

### EXAMPLE 1

### Mutant Hyper-thermostable ssDNA/RNA Ligases

First, a series of hyper-thermostable RNA ligases were identified in a search of the art. This list, in Table 2 below, includes hyperthermophilic archaea species, some of which can endure environments with over 100°C.

**Table 2**

| Genbank ID | Species name | Growth temperature range (°C) | Optimal growth temperature (°C) | IDs used in this application |
|---|---|---|---|---|
| WP_011250496.1 | *Thermococcus kodakarensis* | 65-100 | 85 | TkoRnl2 |
| WP_013906275.1 | *Pyrococcus yayanosii* | 80-108 | 98 | PyaRnl2 |
| WP_010884602.1 | *Pyrococcus horikoshii* | 88-104 | 98 | PhoRnl2 |
| WP_048147341.1 | *Pyrococcus abyssi* * | 90-102 | 96 | Pab1020 |
| WP_014835129.1 | *Pyrococcus furiosus* | 70-103 | 100 | PfuRnl2 |
| WP_011822689.1 | *Hyperthermus butylicus* | Up to 112 | 95-107 | HbuRnl2 |
| BAA80567.2 | *Aeropyrum pernix* | 70-100 | 90-95 | AperRnl2 |
| WP_011839720.1 | *Staphylothermus marinus* | Up to 98 | 85-92 | SmarRnl2 |
| WP_014027000.1 | *Pyrolobusfumarii* | Up to 113 | | PfumRnl2 |
| WP_010880692.1 | *Aquifex aeolicus* | 85-95 | | AaeRnl2 |

| | | | | |
|---|---|---|---|---|
| *Pab1020 has been previously studied and shown to have ssRNA ligation activity, but no ssDNA ligation activity (Brooks et al, Protein Science, 2008). | | | | |

Three of the 10 ligases from Table 2 (PhoRnl2, PfuRnl2, HbuRnl2) were synthesized using optimized *E. coli* codon, expressed and purified, and were examined for their activity on single-strand RNA and DNA substrate at high temperature (90°C as in Figure 1). As shown in Figure 1, the enzymes' activities were assayed under a titration of ATP concentration using 5'-phosphorylated ssRNA and 5'-phosphorylated ssDNA oligonucleotides as substrates. As shown in Figure 1A, 1C and IE, all enzymes tested can turn 5'-phosphorylated ssRNA to 5'-adenylated ssRNA and circularized ssRNA. Under higher concentration of ATP, the ligation reactions appear to stall after the adenyltransfer step (step 2), so that more adenylated products were accumulated. Due to the proximity of the ends in solution within the same ssRNA molecule, intra-molecule ligation is much more preferred than the inter-molecule ligation, so that the major ligation product is the circularized ssRNA. This evidence support that all enzymes are active hyperthermostable ssRNA ligases.

For activity on ssDNA, however, although all enzymes can turn 5'-phosphorylated ssDNA to 5'-adenylated ssDNA, as shown in Figure 1B, 1D and 1F, no circularized ssDNA is formed under varying concentration of ATP, suggesting that they do not possess ssDNA ligation activity, which is also consistent with the Pab1020 study (Brooks et al, Protein Science, 2008). Accumulation of 5'-adenylated ssDNA is higher under higher concentration of ATP, suggesting a similar stall after the step 2 of the adentltransfer. In the case of PfuRnl2, when the ATP concentration is higher than 10uM, it is able to adenylate over 90% of the 5'-phosphorylated ends of ssDNA. The other two enzymes exhibit much lower adenylation efficiency under the same condition (Figure 1). This unique property makes wild-type PfuRnl2 a useful hyperthermostable 5'-adenylase for ssRNA and ssDNA. While the present invention is not limited to any particular mechanism, and an understanding of the mechanism is not necessary to practice the invention, based on these results, it is believed that, although these enzymes can perform the step 2 of adenyltransfer on the 5'-phosphorylated ends of ssDNA, they cannot accomplish the final step of the ligation on ssDNA. In other words, they are not ssDNA ligases in nature.

Next, one of the RNA ligases (HbuRnl2) was tested for its activity on a 5'-adenylated ssDNA under conditions with or without ATP. As shown in Figure 2, wild-type HbuRnl2 can efficiently de-adenylate the 5'-adenylated ssDNA in the absence of ATP (lane 2). The de-adenylation is the reverse process of the step 2 in the ligation reaction, in which the un-adenylated enzyme transfers the AMP back to its catalytic lysine from the 5'-adenylated ends. In the presence of high concentration ATP, this reverse step 2 reaction does not occur (Figure 2, lane 1). While the present invention is not limited to any particular mechanism, and an understanding of the mechanism is not necessary to practice the invention, by combining the observation that HbuRnl2, PfuRnl2, and PhoRnl2 can adenylate a varying proportion of 5'-phosphorylated ssDNA under a titration of ATP, it was hypothesized that the reverse step 2 reaction may block the ligation reaction from moving forward. Reducing the reverse step 2 reaction may push the reaction forward to finish the step 3 of the ligation on ssDNA.

In step 2 of the ligation, the adenyl group is transferred from the catalytic lysine in the conserved Motif I in ligases to the 5'-phosphorylated end. Catalytic lysine resides in a conserved motif EKx(D/N/H)G (SEQ ID NO:32). Thus, while the present invention is not limited to any particular mechanism, and an understanding of the mechanism is not necessary to practice the present invention, by mutating the catalytic lysine in Motif I to other amino acids, the step 2 reverse reaction may be blocked or reduced, so that the step 3 ligation, which is the joining between the 3'-OH end and the 5'-adenylated end, can occur.

Next, HbuRnl2 was chosen as a target, and mutant HbuRn12K106A (SEQ ID NO:7) was constructed and purified, and its activity was assayed in step 3 ligation. As shown in Figure 3, HbuRn12K106A can catalyze the intra-molecule ligation by turning 5'-adenylated ssDNA into circularized ssDNA. After reaction, the circularized ssDNA is resistant to Exonuclease I digestion and was retained on gel after ExoI treatment (Figure 3, lane 2). As a control and comparison, the ligation reaction catalyzed by the wild-type MthRnl at 65°C was also ran and the results revealed the same circularized ssDNA (Figure 3, lanes 3 and 4). Overall, these results confirm that although the wild-type HbuRnl2 does not have the step 3 ligation activity of ssDNA, by mutating the catalytic lysine in HbuRnl2, the enzyme acquires a new activity of ligating 5'-adenylated end and 3'-OH end of ssDNA.

Next, lysine 106 in HbuRnl2 was changed to other amino acids, and mutants were assayed for its adenylated ssDNA circularization activity. As shown in Figure 4, mutants K106T, K106G, K106V, K106C, K106S are active in adenylated ssDNA circularization as well as K106A. Other lysine mutants are inactive in adenylated ssDNA circularization.

The optimal reaction temperature range for HbuRnl2K106A was then examined. As shown in Figure 5, the 5'-adenylated ssDNA circularization activity was assayed in a range of temperature from 70 to 95°C. The activity appears to increase moderately from 70 to 90°C, and reach a plateau after 90°C. For this reason, biochemical assays were conducted at 90°C. This observation is also consistent with the enzyme's origin from a hyperthemophilic archaea and indicates that mutation does not alter its hyperthermostability.

Due to the intrinsic flexibility of ssDNA/RNA and the proximity of the ends of the same oligonucleotide, intra-molecule circularization by ligation occurs in a much higher frequency than inter-molecule concatenation. This may be part of the reason that TS2126 ligase was named "CircLigase" (WO2004/027056A2). Circularization of ssDNA/RNA can be useful for a lot of applications. For example, the circularized oligonucleotides are resistant to exonuclease digestion and can serve as templates for amplification, such as rolling circle amplification. Oftentimes, however, there is a desire to promote inter-molecule ssDNA/RNA ligation instead of intra-molecule circularization, for example, when one wants to attach an oligonucleotide adaptor to a library of ssDNA/RNA.

A number of strategies can be used to promote inter-molecule ligation between ssDNA/RNA. For example, 3'-OH ends of ssDNA/RNA can be modified, such as by using NH2 group or many other modification groups, so that the OH group is not present for the intra-molecule ligation to occur. In addition, Mn²⁺ and ligation enhancer, such as PEG8000, may be used (Zhelkovsky A., et al, BMC Biotechnology, 2012).

In Figure 6, it was shown that by using a molar excess of 5'-adenylated, 3'-NH₂ modified adaptor, inter-molecule ligation is not only possible, but also can approach efficiently to >90% completion for HbuRnl2K106A. Such property is useful, for example, in adaptor ligation in many applications.

Per the above, it was determined that mutant HbuRn12K106A can catalyze the ligation between two pieces of ssRNA, in which both ligation donor and acceptor are RNA, and it can catalyze the ligation between two pieces of ssDNA, in which both ligation donor and acceptor are DNA. It is further demonstrated that it can ligate between ssRNA donor and ssDNA acceptor, or between ssDNA donor and ssRNA acceptor (data not shown). These properties are useful for many applications. For example, current protocols for small RNA cloning and sequencing uses T4Rnl2 and its mutants at 37°C or lower for the attachment of 5'-adenylated ssDNA adaptor to the 3'-OH of ssRNA. One could use HbuRn12K106A for the same purpose but at a much higher temperature with the advantage of minimizing secondary structure.

Per the above, it was shown that PfuRnl2 can efficiently adenylate 5 '-phosphorylated ends of ssDNA (Figure 1). As shown in Figure 8, one can also mix PfuRnl2 and HbuRn12K106A in one-pot reaction so that PfuRnl2 can adenylate the 5'-phosphorylated ends and HbuRn12K106A can join the 5'-adenylated ends with 3'-OH ends of ssDNA. Thus, the enzyme mixture can perform step 1 to step 3 of the ligation reaction with a high reaction temperature.

### Methods and Materials for Example 1

### 1. Synthesis, mutagenesis, expression and purification of hyperthermostable single-stranded RNA/DNA ligases

All genes are synthesized as double-stranded DNA fragment with optimized *E. coli* codon usage and 6X-His-tag at the N-terminus by IDT (Integrated DNA Technologies, Coralville, Iowa). The DNA was then inserted into expression vectors pTXB1 under the control of T7 promoter using Gibson Assembly (NEB #E2611). Expression constructs were transformed into T7 express *E. coli* strain (NEB #C2566). For expression in culture, the cells were grown in LB media to OD ∼0.6, upon which a final concentration of 0.5mM IPTG was added. The induced cultures were kept grown by shaking at 20°C for overnight.

For purification, 250ml of induced cell culture was first pelleted by centrifugation. The cell pellet was then re-suspended in buffer containing 20mM Tris (pH=7.5), 150mM NaCl, 1x FastBreak (Promega), 200ug/ml lysozyme, and sonicated. Cell lysates were then centrifuged at 15,000g for 20min at 4°C. The cleared lysate is then purified successively on a Ni-NTA column and a Heparin column.

Site-directed mutagenesis (e.g., by using New England Biolabs' Q5 mutagenesis kit) was performed with the cloned constructs to introduce mutation to the conserved lysine residue.

### EXAMPLE 2

### Single Stranded Adapter Ligation

Hyperthermostable ssDNA/RNA ligases (e.g., as described in SEQ ID NOS:1-11) can be used in the library preparation process for next-generation high-throughput sequencing. Currently, the dominant library construction methods include a ligation step where double-stranded library fragments are ligated to double-stranded adaptors. Single-strand ligation based method exists, but by using the CircLigase with a optimal reaction temperature around 65°C (Gansauge MT, Nat. Protol., 2013).

As illustrated in Figure 7A, large DNA is first fragmented, for example, by using enzymatic method or by mechanic/ultrasound shearing. Depending on the fragmentation method, the ends of the fragments may require a repairing step using, for example, T4 polynucleotide kinase. The fragmented DNA is then ligated to the first single-strand DNA adaptor, with 5'-adenylated end and 3'-NH₂ end. This inter-molecule ligation is catalyzed by one of the ssDNA/RNA ligases disclosed herein, such as HbuRnl2K106A, which may be in the presence of Mn2+, and PEG8000 at high temperature.

After ligation, a cleanup, *e.g.*, by using AMPure beads or other methods well known in the art, is used to recover all fragments ligated with the first adaptor. The recovered fragments are then adenylated, for example, by using the hyperthermostable 5'-adenylase PfuRnl2 in high concentration of ATP. Then, the 5'-adenylated DNA fragments are ligated with the second adaptor, for example, with regular 5'-OH and 3'-OH ends. This ligation reaction is catalyzed by one of the mutant ligases herein, such as the hyperthermostable HbuRnl2K106A. After ligation, the ligated DNA fragments are purified and ready for further downstream steps, such as via polymerase chain reaction (PCR) or cluster generation.

A number of advantages for a single-strand ligation based library construction exist with the presently disclosed methods, compositions, and systems. First, workflow of NGS sample preparation can be simplified, without end polishing steps. Second, compared with previous protocols, much less bias may exist, especially for long stretches of oligonucleotides in the library, due to high reaction temperature. Third, sequenced reads may be concatenated to form synthetic long reads and assigned to different starting genomic DNA molecules, with the assumption of random breakpoints introduced by the shearing process and relatively deep sequencing coverage so that most of the ssDNA fragments are sequenced (Figure 7B).

### REFERENCES

Blondal et al., Isolation and characterization of a thermostable RNA ligase 1 from a Thermus scotoductus bacteriophage TS2126 with good single-stranded DNA ligation properties. Nucleic Acids Res. 2005 Jan 7;33(1):135-42.
Blondal et al., Discovery and characterization of a thermostable bacteriophage RNA ligase homologous to T4 RNA ligase 1. Nucleic Acids Res. 2003, 31(24):7247-54.
Brooks et al., The structure of an archaeal homodimeric ligase which has RNA circularization activity. Protein Sci. 2008, 17(8):1336-45.
Gansauge and Meyer, Single-stranded DNA library preparation for the sequencing of ancient or damaged DNA. Nat Protoc. 2013, 8(4):737-48.
Ho and Shuman, Bacteriophage T4 RNA ligase 2 (gp24.1) exemplifies a family of RNA ligases found in all phylogenetic domains. Proc Natl Acad Sci USA. 2002, 99(20):12709-14.
Lau et al., An abundant class of tiny RNAs with probable regulatory roles in Caenorhabditis elegans. Science. 2001, 294(5543):858-62.
Lehman IR, DNA ligase: structure, mechanism, and function. Science. 1974 Nov 29;186(4166):790-7.
Lindahl and Barnes, Mammalian DNA ligases. Annu Rev Biochem. 1992;61:251-81.
Silber et al., Purification and properties of bacteriophage T4-induced RNA ligase, Proceedings of the National Academy of Sciences of the United States of America, vol. 69, no. 10, pp. 3009-3013, 1972.
Tomkinson et al., T. DNA ligases: structure, reaction mechanism, and function. Chem Rev. 2006, 106(2):687-99.
Torchia et al., Archaeal RNA ligase is a homodimeric protein that catalyzes intramolecular ligation of single-stranded RNA and DNA. Nucleic Acids Res. 2008, 36(19):6218-27.
US2014/0128292 A1 Methods for improving ligation steps to minimize bias during production of libraries for massively parallel sequencing
Viollet et al., T4 RNA ligase 2 truncated active site mutants: improved tools for RNA analysis. BMC Biotechnol. 2011, 11:72.
WO2004/027056A2 Methods of use for thermostable RNA ligases
Yin et al., Structure-function analysis of T4 RNA ligase 2. J Biol Chem. 2003, 278(20):17601-8
Zhuang et al., Structural bias in T4 RNA ligase-mediated 3'-adapter ligation. Nucleic Acids Res. 2012, 40(7):e54.
Zhelkovsky and McReynolds, Simple and efficient synthesis of 5' pre-adenylated DNA using thermostable RNA ligase. Nucleic Acids Res. 2011, 39(17):e117.
Zhelkovsky and McReynolds, Structure-function analysis of Methanobacterium thermoautotrophicum RNA ligase - engineering a thermostable ATP independent enzyme. BMC Mol Biol. 2012, 13:24.

## Claims

1. A method of ligating single-stranded nucleic acid comprising:
a) combining in a reaction mixture:
i) a hyper-thermostable lysine-mutant ssDNA/RNA ligase which is a mutated version of a precursor hyper-thermostable ssRNA ligase,
wherein said precursor hyper-thermostable ssRNA ligase has a Motif I EKx(D/N/H)G (SEQ ID NO:32) and possess ssRNA ligase activity, but not ssDNA ligase activity, at a temperature of at least 75°C,
ii) a first single stranded nucleic acid sequence with a 5' end base that is adenylated, and
iii) a second single stranded nucleic acid sequence with a 3' end base; and
b) incubating said reaction mixture at a temperature of at least 75°C such that said hyper-thermostable lysine-mutant ssDNA/RNA ligase ligates said 5' adenylated end of said first single stranded nucleic acid sequence to said 3' end of said second single stranded nucleic acid sequence to form a ligated nucleic acid sequence, **characterized in that**
said hyper-thermostable lysine-mutant ssDNA/RNA ligase
- has an amino acid substitution at said K (lysine) in said Motif I, wherein said amino acid substitution for said K (lysine) is an amino acid selected from the group consisting of: serine (S), cysteine (C), valine (V), threonine (T), and glycine (G), and
- possesses both ssRNA ligase and ssDNA ligase activity at a temperature of at least 75°C.

2. The method of Claim 1, wherein said 5' end base of said first single stranded nucleic acid sequence is a DNA base, and wherein said 3' end base of said second single stranded nucleic acid sequence is an RNA base or a DNA base.

3. The method of Claim 1, wherein said 5' end base of said first single stranded nucleic acid sequence is a DNA base or an RNA base, and wherein said 3' end base of said second single stranded nucleic acid sequence is a DNA base.

4. The method of Claim 1, wherein said precursor hyper-thermostable ssRNA ligase is a wild-type hyper-thermostable ssRNA ligase, wherein said wild-type hyper-thermostable ssRNA ligase is preferably from a species selected from the group consisting of: *Thermococcus kodakarensis, Pyrococcus, yayanosii, Pyrococcus horikoshii, Pyrococcus abyssi, Pyrococcus furiosus, Hyperthermus butylicus, Aeropyrum pernix, Staphylothermus marinus, Pyrolobus fumarii, and Aquifex aeolicus.*

5. The method of Claim 1, wherein said hyper-thermostable lysine-mutant ssDNA/RNA ligase is encoded by one of the amino acid sequences shown in SEQ ID NO:1-6 and 8-11.

6. The method of Claim 1, wherein said incubating said reaction mixture is at a temperature of at least 85°C, preferably at a temperature of at least 95°C.

7. The method of Claim 1, wherein said first single stranded nucleic acid sequence is a sequencing adapter.

8. The method of Claim 1, wherein said second single stranded nucleic acid sequence comprises a sequencing library fragment.

9. A system or kit comprising:
a) a hyper-thermostable lysine-mutant ssDNA/RNA ligase which is a mutated version of a precursor hyper-thermostable ssRNA ligase,
wherein said precursor hyper-thermostable ssRNA ligase has a Motif I EKx(D/N/H)G (SEQ ID NO:32) and possess ssRNA ligase activity, but not ssDNA ligase activity, at a temperature of at least 75°C,
b) a first single stranded nucleic acid sequence with a 5' end base that is adenylated; and
c) a second single stranded nucleic acid sequence with a 3' end base, **characterized in that**
said hyper-thermostable lysine-mutant ssDNA/RNA ligase
- has an amino acid substitution at said K (lysine) in said Motif I, wherein said amino acid substitution for said K (lysine) is an amino acid selected from the group consisting of: serine (S), cysteine (C), valine (V), threonine (T), and glycine (G), and
- possesses both ssRNA ligase and ssDNA ligase activity at a temperature of at least 75°C.

10. The system of Claim 9, further comprising a first container and a second container, and wherein said hyper-thermostable lysine-mutant ssDNA/RNA ligase is in said first container, and said first and/or second single-stranded nucleic acid sequence is in said second container.

11. The system of Claim 9, wherein said 5' end base of said first single stranded nucleic acid sequence is a DNA base, and wherein said 3' end base of said second single stranded nucleic acid sequence is an RNA base or a DNA base.

12. A composition comprising: a hyper-thermostable lysine-mutant ssDNA/RNA ligase which is a mutated version of a precursor hyper-thermostable ssRNA ligase,
wherein said precursor hyper-thermostable ssRNA ligase has a Motif I EKx(D/N/H)G (SEQ ID NO:32) and possess ssRNA ligase activity, but not ssDNA ligase activity, at a temperature of at least 75°C,
**characterized in that**
said hyper-thermostable lysine-mutant ssDNA/RNA ligase
- has an amino acid substitution at said K (lysine) in said Motif I, wherein said amino acid substitution for said K (lysine) is an amino acid selected from the group consisting of: serine (S), cysteine (C), valine (V), threonine (T), and glycine (G), and
- possesses both ssRNA ligase and ssDNA ligase activity at a temperature of at least 75°C.

13. The composition of Claim 12, further comprising at least one of the following:
a) a first single stranded nucleic acid sequence with a 5' end base that is adenylated, and
b) a second single stranded nucleic acid sequence with a 3' end base.

## Patentansprüche

1. Verfahren zum Ligieren von einzelsträngiger Nukleinsäure, umfassend:
a) Kombinieren in einem Reaktionsgemisch:
i) eine hyper-thermostabile Lysin-mutante ssDNA/RNA-Ligase, die eine mutierte Version einer Vorläufer-hyper-thermostabilen ssRNA-Ligase ist, wobei die Vorläufer-hyper-thermostabile ssRNA-Ligase ein Motiv I EKx(D/N/H)G (SEQ ID Nr.:32) hat und ssRNA-Ligase-Aktivität, aber nicht ssDNA-Ligase-Aktivität, bei einer Temperatur von mindestens 75°C besitzt,
ii) eine erste einzelsträngige Nukleinsäuresequenz mit einer 5'-Endbase, die adenyliert ist, und
iii) eine zweite einzelsträngige Nukleinsäuresequenz mit einer 3'-Endbase; und
b) Inkubieren des Reaktionsgemisches bei einer Temperatur von mindestens 75°C, so dass die hyper-thermostabile Lysin-mutante ssDNA/RNA-Ligase das 5'-adenylierte Ende der ersten einzelsträngigen Nukleinsäuresequenz an das 3'-Ende der zweiten einzelsträngigen Nukleinsäuresequenz unter Bildung einer ligierten Nukleinsäuresequenz ligiert,
**dadurch gekennzeichnet, dass**
besagte hyper-thermostabile Lysin-mutante ssDNA/RNA-Ligase
- eine Aminosäuresubstitution an dem K (Lysin) in Motiv I aufweist, wobei die Aminosäuresubstitution für das K (Lysin) eine Aminosäure ist, ausgewählt aus der Gruppe bestehend aus: Serin (S), Cystein (C), Valin (V), Threonin (T) und Glycin (G), und
- beides besitzt, sowohl ssRNA-Ligase-Aktivität als auch ssDNA-Ligase-Aktivität bei einer Temperatur von mindestens 75°C.

2. Verfahren nach Anspruch 1, wobei die 5'-Endbase der ersten einzelsträngigen Nukleinsäuresequenz eine DNA-Base ist, und wobei die 3'-Endbase der zweiten einzelsträngigen Nukleinsäuresequenz eine RNA-Base oder eine DNA-Base ist.

3. Verfahren nach Anspruch 1, wobei die 5'-Endbase der ersten einzelsträngigen Nukleinsäuresequenz eine DNA-Base oder eine RNA-Base ist, und wobei die 3'-Endbase der zweiten einzelsträngigen Nukleinsäuresequenz eine DNA-Base ist.

4. Verfahren nach Anspruch 1, wobei die Vorläufer-hyper-thermostabile ssRNA-Ligase eine Wildtyp-hyper-thermostabile ssRNA-Ligase ist, wobei die Wildtyp-hyper-thermostabile ssRNA-Ligase vorzugsweise aus einer Spezies ist, ausgewählt aus der Gruppe bestehend aus: *Thermococcus kodakarensis, Pyrococcus, yayanosii, Pyrococcus horikoshii, Pyrococcus abyssi, Pyrococcus furiosus, Hyperthermus butylicus, Aeropyrum pernix, Staphylothermus marinus, Pyrolobus fumarii,* und *Aquifex aeolicus.*

5. Verfahren nach Anspruch 1, wobei die hyper-thermostabile Lysin-mutante ssDNA/RNA-Ligase durch eine der in SEQ ID Nr.:1-6 und 8-11 gezeigten Aminosäuresequenzen kodiert wird.

6. Verfahren nach Anspruch 1, wobei das Inkubieren der Reaktionsmischung bei einer Temperatur von mindestens 85°C, vorzugsweise bei einer Temperatur von mindestens 95°C, erfolgt.

7. Verfahren nach Anspruch 1, wobei die erste einzelsträngige Nukleinsäuresequenz ein Sequenzierungsadapter ist.

8. Verfahren nach Anspruch 1, wobei die zweite einzelsträngige Nukleinsäuresequenz ein Sequenzierungsbibliotheksfragment umfasst.

9. System oder Kit umfassend:
a) eine hyper-thermostabile Lysin-mutante ssDNA/RNA-Ligase, die eine mutierte Version einer Vorläufer-hyper-thermostabilen ssRNA- Ligase ist,
wobei die Vorläufer-hyper-thermostabile ssRNA-Ligase ein Motiv I EKx(D/N/H)G (SEQ ID Nr.:32) hat und ssRNA-Ligase-Aktivität, aber nicht ssDNA-Ligase-Aktivität, bei einer Temperatur von mindestens 75°C besitzt,
b) eine erste einzelsträngige Nukleinsäuresequenz mit einer 5'-Endbase, die adenyliert ist; und
c) eine zweite einzelsträngige Nukleinsäuresequenz mit einer 3'-Endbase,
**dadurch gekennzeichnet, dass**
besagte hyper-thermostabile Lysin-mutante ssDNA/RNA-Ligase
- eine Aminosäuresubstitution an dem K (Lysin) in Motiv I aufweist, wobei die Aminosäuresubstitution für das K (Lysin) eine Aminosäure ist, ausgewählt aus der Gruppe bestehend aus: Serin (S), Cystein (C), Valin (V), Threonin (T) und Glycin (G), und
- beides besitzt, sowohl ssRNA-Ligase-Aktivität als auch ssDNA-Ligase-Aktivität bei einer Temperatur von mindestens 75°C.

10. System nach Anspruch 9, das des Weiteren einen ersten Behälter und einen zweiten Behälter umfasst, und wobei sich die hyperthermostabile Lysin-mutante ssDNA/RNA-Ligase in dem ersten Behälter befindet und sich die erste und/oder zweite einzelsträngige Nukleinsäuresequenz in dem zweiten Behälter befindet.

11. System nach Anspruch 9, wobei die 5'-Endbase der ersten einzelsträngigen Nukleinsäuresequenz eine DNA-Base ist, und wobei die 3'-Endbase der zweiten einzelsträngigen Nukleinsäuresequenz eine RNA-Base oder eine DNA-Base ist.

12. Zusammensetzung, umfassend: eine hyper-thermostabile Lysin-mutante ssDNA/RNA-Ligase, die eine mutierte Version einer Vorläufer-hyper-thermostabilen ssRNA-Ligase ist,
wobei die Vorläufer-hyper-thermostabile ssRNA-Ligase ein Motiv I
EKx(D/N/H)G (SEQ ID Nr.:32) hat und ssRNA-Ligase-Aktivität, aber nicht ssDNA-Ligase-Aktivität, bei einer Temperatur von mindestens 75°C besitzt,
**dadurch gekennzeichnet, dass**
besagte hyper-thermostabile Lysin-mutante ssDNA/RNA-Ligase
- eine Aminosäuresubstitution an dem K (Lysin) in Motiv I aufweist, wobei die Aminosäuresubstitution für das K (Lysin) eine Aminosäure ist, ausgewählt aus der Gruppe bestehend aus: Serin (S), Cystein (C), Valin (V), Threonin (T) und Glycin (G), und
- beides besitzt, sowohl ssRNA-Ligase-Aktivität als auch ssDNA-Ligase-Aktivität bei einer Temperatur von mindestens 75°C.

13. Zusammensetzung nach Anspruch 12, die des Weiteren mindestens eines der folgenden umfasst:
a) eine erste einzelsträngige Nukleinsäuresequenz mit einer 5'-Endbase, die adenyliert ist, und
b) eine zweite einzelsträngige Nukleinsäuresequenz mit einer 3'-Endbase.

## Revendications

1. Procédé de ligature d'un acide nucléique en simple brin, comprenant les étapes consistant à :
a) combiner dans un mélange réactionnel :
i) une ADN/ARNss ligase hyper-thermostable mutante pour une lysine qui est une version mutée d'une ARNss ligase hyper-thermostable précurseur,
dans lequel ladite ARNss ligase hyper-thermostable précurseur a un Motif I EKx(D/N/H)G (SEQ ID n° : 32) et possède une activité d'ARNss ligase, mais pas d'activité d'ADNss ligase, à une température d'au moins 75°C,
ii) une première séquence d'acides nucléiques en simple brin avec une base à l'extrémité 5' qui est adénylée et
iii) une deuxième séquence d'acides nucléiques en simple brin avec une base à l'extrémité 3' ; et
b) incuber ledit mélange réactionnel à une température d'au moins 75°C de telle sorte que ladite ADN/ARNss ligase hyper-thermostable mutante pour une lysine ligature ladite extrémité 5' adénylée de ladite première séquence d'acides nucléiques en simple brin à ladite extrémité 3' de ladite deuxième séquence d'acides nucléiques en simple brin pour former une séquence d'acides nucléiques ligaturés,
**caractérisé en ce que**
ladite ADN/ARNss ligase hyper-thermostable mutante pour une lysine
- a une substitution d'acide aminé au niveau dudit K (lysine) dans ledit Motif I, ladite substitution d'acide aminé pour ledit K (lysine) étant un acide aminé choisi dans le groupe constitué par les : sérine (S), cystéine (C), valine (V), thréonine (T) et glycine (G),
et
- possède des activités à la fois d'ARNss ligase et d'ADNss ligase à une température d'au moins 75°C.

2. Procédé de la revendication 1, dans lequel ladite base à l'extrémité 5' de ladite première séquence d'acides nucléiques en simple brin est une base d'ADN et dans lequel ladite base à l'extrémité 3' de ladite deuxième séquence d'acides nucléiques en simple brin est une base d'ARN ou une base d'ADN.

3. Procédé de la revendication 1, dans lequel ladite base à l'extrémité 5' de ladite première séquence d'acides nucléiques en simple brin est une base d'ADN ou une base d'ARN et dans lequel ladite base à l'extrémité 3' de ladite deuxième séquence d'acides nucléiques en simple brin est une base d'ADN.

4. Procédé de la revendication 1, dans lequel ladite ARNss ligase hyper-thermostable précurseur est une ARNss ligase hyper-thermostable de type sauvage, dans lequel ladite ARNss ligase hyper-thermostable de type sauvage provient, de préférence, d'une espèce choisie dans le groupe constitué par : *Thermococcus kodakarensis*, *Pyrococcus*, *yayanosii*, *Pyrococcus horikoshii*, *Pyrococcus abyssi*, *Pyrococcus furiosus*, *Hyperthermus butylicus*, *Aeropyrum pernix*, *Staphylothermus marinus*, *Pyrolobus fumarii et Aquifex aeolicus.*

5. Procédé de la revendication 1, dans lequel ladite ADN/ARNss ligase hyper-thermostable mutante pour une lysine est codée par l'une des séquences d'acides aminés présentées dans les SEQ ID n° : 1 à 6 et 8 à 11.

6. Procédé de la revendication 1, dans lequel ladite incubation dudit mélange réactionnel se fait à une température d'au moins 85°C, de préférence à une température d'au moins 95°C.

7. Procédé de la revendication 1, dans lequel ladite première séquence d'acides nucléiques en simple brin est un adaptateur de séquençage.

8. Procédé de la revendication 1, dans lequel ladite deuxième séquence d'acides nucléiques en simple brin comprend un fragment de banque de séquençage.

9. Système ou trousse comprenant :
a) une ADN/ARNss ligase hyper-thermostable mutante pour une lysine qui est une version mutée d'une ARNss ligase hyper-thermostable précurseur,
dans lequel ladite ARNss ligase hyper-thermostable précurseur a un Motif I EKx(D/N/H)G (SEQ ID n° : 32) et possède une activité d'ARNss ligase, mais pas d'activité d'ADNss ligase, à une température d'au moins 75°C,
b) une première séquence d'acides nucléiques en simple brin avec une base à l'extrémité 5' qui est adénylée ; et
c) une deuxième séquence d'acides nucléiques en simple brin avec une base à l'extrémité 3',
**caractérisé en ce que**
ladite ADN/ARNss ligase hyper-thermostable mutante pour une lysine
- a une substitution d'acide aminé au niveau dudit K (lysine) dans ledit Motif I, ladite substitution d'acide aminé pour ledit K (lysine) étant un acide aminé choisi dans le groupe constitué par les : sérine (S), cystéine (C), valine (V), thréonine (T) et glycine (G),
et
- possède des activités à la fois d'ARNss ligase et d'ADNss ligase à une température d'au moins 75°C.

10. Système de la revendication 9, comprenant en outre un premier conteneur et un deuxième conteneur, et dans lequel ladite ADN/ARNss ligase hyper-thermostable mutante pour une lysine est dans ledit premier conteneur et ladite première et/ou ladite deuxième séquence d'acides nucléiques en simple brin est/sont dans ledit deuxième conteneur.

11. Système de la revendication 9, dans lequel ladite base à l'extrémité 5' de ladite première séquence d'acides nucléiques en simple brin est une base d'ADN et dans lequel ladite base à l'extrémité 3' de ladite deuxième séquence d'acides nucléiques en simple brin est une base d'ARN ou une base d'ADN.

12. Composition comprenant : une ADN/ARNss ligase hyper-thermostable mutante pour une lysine qui est une version mutée d'une ARNss ligase hyper-thermostable précurseur,
dans laquelle ladite ARNss ligase hyper-thermostable précurseur a un Motif I EKx(D/N/H)G (SEQ ID n° : 32) et possède une activité d'ARNss ligase, mais pas d'activité d'ADNss ligase, à une température d'au moins 75°C,
**caractérisée en ce que**
ladite ADN/ARNss ligase hyper-thermostable mutante pour une lysine
- a une substitution d'acide aminé au niveau dudit K (lysine) dans ledit Motif I, ladite substitution d'acide aminé pour ledit K (lysine) étant un acide aminé choisi dans le groupe constitué par les : sérine (S), cystéine (C), valine (V), thréonine (T) et glycine (G), et
- possède des activités à la fois d'ARNss ligase et d'ADNss ligase à une température d'au moins 75°C.

13. Composition de la revendication 12, comprenant en outre au moins l'un des éléments suivants :
a) une première séquence d'acides nucléiques en simple brin avec une base à l'extrémité 5' qui est adénylée et
b) une deuxième séquence d'acides nucléiques en simple brin avec une base à l'extrémité 3'.
